# EUROPEAN PATENT SPECIFICATION

(11) **EP 4 148 114 B1**
(45) Date of publication and mention of the grant of the patent: **28.01.2026**
(21) Application number: 21195867.3
(22) Date of filing: 10.09.2021
(51) Int. Cl.: C12M 1/32, C12M 1/00, C12M 1/12

(54) **CELL CULTURE APPARATUS, METHODS FOR CELL CULTIVATION BY USING THE SAME, AND CELL CULTURE INCUBATOR COMPRISING THE SAME**
ZELLKULTURVORRICHTUNG, VERFAHREN ZUR KULTIVIERUNG VON ZELLEN UNTER VERWENDUNG DERSELBEN UND ZELLKULTURINKUBATOR DAMIT
APPAREIL DE CULTURE CELLULAIRE, PROCÉDÉS DE CULTURE CELLULAIRE L'UTILISANT ET INCUBATEUR DE CULTURE CELLULAIRE LE COMPRENANT

(43) Date of publication of application: 15.03.2023
(73) Proprietor: Finnadvance Oy, 90220 Oulu (FI)
(72) Inventor: Singh, Prateek, 90130 Oulu (FI); Nguyen, Tuan, 90130 Oulu (FI); Nurmi, Tuomas, 90240 Oulu (FI); Kihlström, Minna, 90420 Oulu (FI)
(74) Representative: Papula Oy

(56) References cited:
- WO-A1-2020/081740
- WO-A1-2020/109421
- AU-B2- 2018 283 184
- US-A1- 2013 059 322

## Description

### TECHNICAL FIELD

The present disclosure relates generally to the field of biotechnology. In particular, the present disclosure relates to a cell culture apparatus, methods for cell cultivation by using the cell culture apparatus, and a cell culture incubator comprising the cell culture apparatus.

### BACKGROUND

Cell culture or cell cultivation involves growing cells of desired type(s) outside a living body under controlled *in vitro* conditions to sustain cell growth and viability. The cell culture is widely used in different biotechnology branches, including cell biology, tissue engineering, biomedical engineering, cell differentiation studies, cell-based biosensors, cell-cell interaction, cell-signaling, cell-migration, physiological and pathophysiological studies, etc.

Environmental conditions created for cultured cells should resemble as closely as possible the conditions experienced by the same cells *in vivo.* This may be done by performing the cell culture in large vessels, such as dishes, spinner and shaker flasks, etc. However, the cell culture conditions provided by these vessels do not truly represent the *in vivo* environment of the cells being cultured. Moreover, since these vessels have a large volume, they consume significant amounts of reagents, culture media, chemicals, etc., thereby making it difficult to control and/or alter the cell culture conditions.

With the advent of microfluidics, new devices and methods configured to cultivate various types of cells, like adherent and non-adherent, under uniform and controlled *in vitro* conditions have been developed. Unlike the conventional cell culture methods based on the above-mentioned vessels, microfluidic cell culture methods may provide continuous nutrient (culture fluid or medium) supply, waste removal, flexibility of schedules, and high automation capability. The less consumption of fluids, their low volumes and therefore the reduced time and cost of cell cultivation make these microfluidic methods particularly interesting for cell-based assays. The main goals of microfluidic cell culture devices are to mimic closely *in vivo* cellular microenvironments and to maintain simplicity for reproducible results.

Patent Application WO 2020/10942 discloses a microfluidic apparatus where the central volume of the unit is flanked by two lateral volumes, but they are in direct fluid contact at their bottom without separation by a membrane with no function of permeability.

However, the limiting factors of the current microfluidic cell culture devices are their low throughput and incompatibility with available liquid handling systems and imaging systems due to the discrete arrangement and limited number of microfluidic channels. Furthermore, fluid flow control in the current microfluidic cell culture devices is often limited to one method and not flexible enough to adapt to different resource settings and different cell-based assays.

### SUMMARY

This summary is provided to introduce a selection of concepts in a simplified form that are further described below in the detailed description. This summary is not intended to identify key features of the present disclosure, nor is it intended to be used to limit the scope of the present disclosure.

It is an objective of the present disclosure to provide a technical solution that enables high-throughput microfluidic cell culture.

The objective above is achieved by the features of the independent claims in the appended claims. Further embodiments and examples are apparent from the dependent claims, the detailed description and the accompanying drawings.

According to a first aspect, a cell culture apparatus is provided. The apparatus comprises a plurality of cell culture modules arranged adjacent to each other. Each cell culture module of the plurality of cell culture modules comprises at least two culture medium reservoirs, a first chamber, a second chamber, a membrane, and at least two flow channels. Each of the at least two culture medium reservoirs has a top part and a bottom part, with the top part having an inlet for a culture medium and the bottom part having an outlet for the culture medium. The first chamber is arranged between the at least two culture medium reservoirs such that the first chamber and the at least two culture medium reservoirs are aligned with each other in a first direction. The second chamber is arranged under the first chamber and aligned with the first chamber in a second direction. The second direction is perpendicular to the first direction. The second chamber has a bottom part having at least two lateral holes. The bottom part of the second chamber is arranged higher than the bottom part of each of the at least two culture medium reservoirs. The membrane has through pores formed therein and is arranged between the first chamber and the second chamber such that the first chamber and the second chamber are in flow communication with each other via the through pores. Each of the at least two flow channels connects the outlet of the bottom part of one of the at least two culture medium reservoirs to one of the at least two lateral holes of the bottom part of the second chamber. The apparatus further comprises a flow driving unit configured to cause the culture medium to flow, in each cell culture module of the plurality of cell culture modules, between the at least two culture medium reservoirs via the at least two flow channels and the second chamber. With this configuration, the cell culture apparatus may cultivate cells of the same or different types on the basal (i.e., bottom) surface of the porous membranes in the cell culture modules under uniform and controlled *in vitro* conditions. Moreover, the deposition of the cells on the basal surface of the porous membranes may be provided without having to invert the apparatus or to use high concentrations of the cells in the culture medium to cause the cells to bond and cover the membrane.

In one embodiment of the first aspect, the first chamber in at least one cell culture module of the plurality of cell culture modules is implemented as a bottomless hollow tube. With this first chamber, it is possible to deposit cells on the apical (i.e., top) surface of the porous membrane. The cells deposited on the apical surface of the porous membrane may be of the same or other type compared to the cells deposited on the basal surface of the porous membrane. Thus, the first chamber thus configured may allow one to deposit a cell monolayer on the apical surface of the porous membrane and study the interaction of the cells deposited on the basal and apical surfaces of the membranes. Moreover, with such configuration, the first chamber is easier and cheaper to manufacture.

In one embodiment of the first aspect, the first chamber in at least one cell culture module of the plurality of cell culture modules is implemented as a hollow tube having a curved or angled bottom. In this embodiment, the curved or angled bottom has at least one cavity and at least one hole formed in each of the at least one cavity. With this first chamber, it is possible to deposit cells on separate portions of the apical surface of the porous membrane.

This configuration of the first chamber is especially useful for forming similar or different particles under study (e.g., organoids or spheroids) on the apical surface of the porous membrane and study their interaction with the cells deposited on the basal surface of the porous membrane.

In one embodiment of the first aspect, the curved or angled bottom is coated, from outside, with a cell adhesion enhancing layer such that the at least one hole of the at least one cavity remains open. The cell adhesion enhancing layer may be made of a hydrophilic material (e.g., type I collagen). With such a coating layer, the cells may clump outside the bottom of the first chamber and further adhere onto the apical surface of the porous membrane. Thus, this coating layer allows for less cells to be used in an experiment and more precise deposition of the cells on the apical surface of the porous membrane. In turn, the smaller number of cells also means less cell death or, in other words, more healthy cells in the cell culture as dead cells tend to release factors promoting apoptosis.

In one embodiment of the first aspect, the second chamber has a height falling within a range from 50 µm to 150 µm. By using such a second chamber, it is possible to make the cell culture apparatus according to the first aspect more compact.

In one embodiment of the first aspect, the second chamber in at least one cell culture module of the plurality of cell culture modules is coated, from inside, with a cell adhesion enhancing layer. The cell adhesion enhancing layer may be made of a hydrophilic material. (e.g., type I collagen). With such a coating layer, the cells may attach onto the basal surface of the porous membrane more efficiently. Thus, this coating layer allows for less cells to be used in an experiment and more precise deposition of the cells on the basal surface of the porous membrane. In turn, the smaller number of cells also means less cell death or, in other words, more healthy cells in the cell culture as dead cells tend to release factors promoting apoptosis.

In one embodiment of the first aspect, each of the at least two flow channels in at least one cell culture module of the plurality of cell culture modules is coated, from inside, with a cell adhesion enhancing layer. The cell adhesion enhancing layer may be made of a hydrophilic material (e.g., type I collagen). By using the adhesion enhancing layer, cell attachment onto the sides of the flow channels (as well as on the sides of the second chamber and the porous membrane) becomes stronger, cell proliferation is appropriate to a tissue being modelled, and cell viability is better. Selecting the correct adhesion enhancing layer also plays a role in the proper communication between cell types. Thus, this coating layer allows for less cells to be used in an experiment and more precise deposition of the cells on the basal surface of the porous membrane. In turn, the smaller number of cells also means less cell death or, in other words, more healthy cells in the cell culture as dead cells tend to release factors promoting apoptosis.

In one embodiment of the first aspect, the flow driving unit is configured to cause the culture medium to flow, in each cell culture module of the plurality of cell culture modules, between the at least two culture medium reservoirs by:
- supplying a compressed gas or a pressurized air to the inlet of each of the at least two culture medium reservoirs; or
- pipetting the culture medium from one of the at least two culture medium reservoirs to another of the at least two culture medium reservoirs at regular time intervals or based on a level of the culture medium in each of the at least two culture medium reservoirs; or
- periodically rocking the plurality of cell culture modules.

Thus, unlike the current microfluidic cell culture devices, the cell culture apparatus according to the first aspect is compatible with different flow control means, i.e., pneumatic pump-actuated flow control, and pumpless flow control (which is provided by means of gravity as a result of rocking the plurality of cell culture modules or by means of culture medium pipetting).

In one embodiment of the first aspect, each of the at least two culture medium reservoirs in at least one cell culture module of the plurality of cell culture modules is implemented as a hollow tube. With such configuration, the culture medium reservoirs are easier and cheaper to manufacture.

In one embodiment of the first aspect, the bottom part of each of the at least two culture medium reservoirs has a positively tapered profile. By using such a configuration of the culture medium reservoirs, it is possible to cause the whole culture medium contained in the culture medium reservoirs to flow to the bottom towards the second chamber even when the apparatus is tilted, so that no culture medium is left in the culture medium reservoirs. This also helps to reduce the number of cells that is needed to be deposited or grown on the basal surface of the porous membrane. Additionally, this configuration of the culture medium reservoirs may allow one to achieve optimal flow rates in each cell culture module.

In one embodiment of the first aspect, at least one cell culture module of the plurality of cell culture modules further comprises a first electrode arranged in the first chamber and a second electrode arranged in the second chamber. These electrodes may serve different purposes. For example, they may be used to feed electrical pulses to muscle or nerve cells present in the first and second chambers or be coupled to other sensors of various types. More specifically, these electrodes may be used to perform real-time Transepithelial/Transendothelial Electrical Resistance (TEER) measurements; in this case, signals may be read out by an external control unit.

In another embodiment of the first aspect, at least one cell culture module of the plurality of cell culture modules further comprises a first electrode arranged in one or more of the at least two culture medium reservoirs and a second electrode arranged in the first chamber. This arrangement of the first and second electrodes may be also used to perform the TEER measurements, for example.

In one embodiment of the first aspect, at least one cell culture module of the plurality of cell culture modules further comprises at least two third electrodes arranged in the first chamber. The third electrodes may be arranged on the same wall or on opposite walls inside the first chamber. These electrodes may be used to perform liquid/medium level measurements in the first chamber.

In one embodiment of the first aspect, at least one cell culture module of the plurality of cell culture modules further comprises at least two fourth electrodes imbedded into the membrane. These electrodes may be used, for example, to provide different stimulus signals to the cells deposited on the apical and/or basal surface of the membrane.

In one embodiment of the first aspect, at least one cell culture module of the plurality of cell culture modules further comprises at least one of an oxygen sensor, a pH sensor and a CO2 sensor in the first chamber or the second chamber. These sensors enable real-time measurements of parameters crucial for cell behavior.

In one embodiment of the first aspect, each of the at least two flow channels extend at least partly at a tilting angle to the first direction. In this embodiment, the tilting angle fall within a range of 5 degrees to 45 degrees. With such titled flow channels, it is possible to achieve appropriate flow characteristics and, therefore, improve cell delivery to the second chamber.

In one embodiment of the first aspect, the through pores of the membrane has an average pore size falling within a range from 0.2 µm to 10 µm. By varying the average pore size within this range, it is possible to study the behavior of cells of different sizes, which are deposited on one or each of the apical and basal surfaces of the membrane.

In one embodiment of the first aspect, each of the at least two flow channels in at least one cell culture module of the plurality of cell culture modules has a variable channel height and a variable channel width which increase towards the second chamber. With this configuration of the flow channels, it is possible to improve flow characteristics in each cell culture module, thereby improving the cell delivery to the second chamber and, consequently, the cell attachment to the basal surface of the porous membrane.

In one embodiment of the first aspect, the variable channel width gradually increases towards the second chamber. This may allow achieving better retainment of the laminar flow of the culture medium in the flow channels.

In one embodiment of the first aspect, each of the at least two flow channels in at least one cell culture module of the plurality of cell culture modules comprises a first channel portion and a second channel portion. The first channel portion extends from the outlet of the bottom part one of the at least two culture medium reservoirs and is parallel to the first direction. The second channel portion connects the first channel portion to one of the at least two lateral holes of the second chamber. The second portion is tilted relative to the first direction. With this configuration of the flow channels, it is possible to improve flow characteristics in each cell culture module, thereby improving the cell delivery to the second chamber.

According to a second aspect, a cell culture incubator is provided. The cell culture incubator comprises the cell culture apparatus according to the first aspect, and a pipetting station configured to feed the culture medium to each of the at least two culture medium reservoirs in each cell culture module of the plurality of cell culture modules. With this configuration, the cell culture incubator may cultivate cells of the same or different types on the porous membranes of the cell culture modules under unform and controlled *in vitro* conditions. Moreover, the deposition of the cells on the basal surface of the porous membranes may be provided without having to invert the cell culture apparatus or to use high concentrations of the cells in the culture medium to cause the cells to bond and cover the membrane.

According to a third aspect, a method for cell cultivation by using the cell culture apparatus according to the first aspect is provided. The method starts with the step of providing the culture medium to one of the at least two medium reservoirs in each cell culture module of the plurality of cell culture modules. Then, the method proceeds to the step of causing, by the flow driving unit, the culture medium to flow from said one of the at least two culture medium reservoirs to the rest of the at least two culture medium reservoirs in each cell culture module of the plurality of cell culture modules for a predefined time period. The predefined time period is selected based on the culture medium. By so doing, it is possible to cultivate cells of the same or different types on the porous membranes of the cell culture modules under unform and controlled *in vitro* conditions. Moreover, the deposition of the cells on the basal surface of the porous membranes may be provided without having to invert the cell culture apparatus or to use high concentrations of the cells in the culture medium to cause the cells to bond and cover the membrane.

In one embodiment of the third aspect, the method further comprises, during the step of causing the culture medium to flow, the step of feeding another culture medium via the first chamber towards the membrane in at least one cell culture module of the plurality of cell culture modules. By so doing, it is possible to cultivate a first type of cells on the basal surface of the porous membranes and a different second type of cells on the apical surface of the porous membranes, thereby generating different cell co-cultures.

In one embodiment of the third aspect, the culture medium is caused to flow by applying a positive pressure to the at least two culture medium reservoirs in each cell culture module of the plurality of cell culture modules for the predefined time period. By so doing, it is possible to provide a proper culture medium flow in each cell culture module, thereby improving cell cultivation on the porous membranes in each cell culture module.

In yet another embodiment of the third aspect, the culture medium is caused to flow by applying a positive pressure to said one of the at least two culture medium reservoirs in each cell culture module of the plurality of cell culture modules for the predefined time period, while capping the rest of the at least two culture medium reservoirs. By so doing, it is possible to provide a proper culture medium flow in each cell culture module, thereby improving cell cultivation on the porous membranes in each cell culture module.

In yet another embodiment of the third aspect, the at least two culture medium reservoirs in each cell culture module of the plurality of cell culture modules comprises a first culture medium reservoir and a second culture medium reservoir. In this embodiment, the culture medium is caused to flow by:
- applying a first positive pressure to the first culture medium reservoir in each cell culture module of the plurality of cell culture modules for the predefined time interval, while capping the second culture medium reservoir; and
- applying a second positive pressure to the second culture medium reservoir in each cell culture module of the plurality of cell culture modules for the predefined time interval, while capping the first culture medium reservoir.

By so doing, it is possible to provide a proper culture medium flow in each cell culture module, thereby improving cell cultivation on the porous membranes in each cell culture module.

According to a fourth aspect, a method for cell cultivation by using the cell culture apparatus according to the first aspect is provided. The method starts with the step of providing the culture medium to one or more of the at least two medium reservoirs in each cell culture module of the plurality of cell culture modules. Then, the method proceeds to the step of causing, by the flow driving unit, the culture medium to flow to the second chamber from said one or more of the at least two medium reservoirs in each cell culture module of the plurality of cell culture modules. After that, the method goes on to the step of placing the cell culture apparatus in an inverted position. Next, the method proceeds to the step of incubating the cell culture apparatus in the inverted position for a predefined time period. The predefined time period is selected based on the culture medium. By so doing, it is possible to cultivate cells of the same or different types on the porous membranes of the cell culture modules under unform and controlled *in vitro* conditions. Although the deposition of the cells on the basal surface of the porous membranes is provided by inverting the cell culture apparatus, the method according to the fourth aspect does not require high concentrations of the cells to be used in the culture medium to cause the cells to bond and cover the membrane.

In one embodiment of the fourth aspect, the method further comprises, during the step of causing the culture medium to flow, the step of feeding another culture medium via the first chamber towards the membrane in at least one cell culture module of the plurality of cell culture modules. By so doing, it is possible to cultivate a first type of cells on the basal surface of the porous membranes and a different second type of cells on the apical surface of the porous membranes, thereby generating different cell co-cultures.

In one embodiment of the fourth aspect, the culture medium comprises human brain vascular endothelial cells and said another culture medium comprises human astrocytes. By using these types of cells, it is possible to create artificial blood-brain barriers (BBBs) or create human mimicking artificial BBBs.

Other features and advantages of the present disclosure will be apparent upon reading the following detailed description and reviewing the accompanying drawings.

### BRIEF DESCRIPTION OF THE DRAWINGS

The present disclosure is explained below with reference to the accompanying drawings in which:
FIG. 1 shows a block diagram of a cell culture apparatus in accordance with one exemplary embodiment;
FIGs. 2A-2C show different schematic views of a cell culture module included in the apparatus shown in FIG. 1 in accordance with a first exemplary embodiment, namely: FIG. 2A shows a schematic perspective view of the cell culture module, FIG. 2B shows a schematic side view of the cell culture module, and FIG. 2C shows a schematic top view of the cell culture module;
FIG. 3 shows a sectional view of one of two flow channels of the cell culture module, as taken within the area delimited by oval A in FIG. 2B;
FIG. 4 shows a schematic exploded view of a layered structure that represents one possible implementation example of the cell culture module shown in FIGs. 2A-2C;
FIGs. 5A-5C show different schematic views of the cell culture module included in the apparatus shown in FIG. 1 in accordance with a second exemplary embodiment, namely: FIG. 5A shows a schematic perspective view of the cell culture module, FIG. 5B shows a schematic side view of the cell culture module, and FIG. 5C shows a schematic top view of the cell culture module;
FIG. 6 shows a block diagram of a cell culture incubator in accordance with one exemplary embodiment;
FIG. 7 shows a flowchart of a method for cell cultivation by using the apparatus shown in FIG. 1 in accordance with a first exemplary embodiment; and

FIG. 8 shows a flowchart of a method for cell cultivation by using the apparatus shown in FIG. 1 in accordance with a second exemplary embodiment; and

FIGs. 9A and 9B show confocal images of a BBB model by using the apparatus shown in FIG. 1 in accordance with the method shown in FIG. 8.

### DETAILED DESCRIPTION

Various embodiments of the present disclosure are further described in more detail with reference to the accompanying drawings. However, the present disclosure may be embodied in many other forms and should not be construed as limited to any certain structure or function discussed in the following description. In contrast, these embodiments are provided to make the description of the present disclosure detailed and complete.

According to the detailed description, it will be apparent to the ones skilled in the art that the scope of the present disclosure encompasses any embodiment thereof, which is disclosed herein, irrespective of whether this embodiment is implemented independently or in concert with any other embodiment of the present disclosure. For example, apparatuses and/or methods disclosed herein may be implemented in practice using any numbers of the embodiments provided herein. Furthermore, it should be understood that any embodiment of the present disclosure may be implemented using one or more of the elements presented in the appended claims.

The word "exemplary" is used herein in the meaning of "used as an illustration". Unless otherwise stated, any embodiment described herein as "exemplary" should not be construed as preferable or having an advantage over other embodiments.

Any positioning terminology, such as "left", "right", "top", "bottom", "above", "under", "apical", "basal", etc., may be used herein for convenience to describe one element's or feature's relationship to one or more other elements or features in accordance with the figures. It should be apparent that the positioning terminology is intended to encompass different orientations of the structure and device disclosed herein, in addition to the orientation(s) depicted in the figures. As an example, if one imaginatively rotates the structure or device in the figures 90 degrees clockwise, elements or features described as "top" and "bottom" relative to other elements or features would then be oriented, respectively, "right" and "left" relative to the other elements or features. Therefore, the positioning terminology used herein should not be construed as any limitation of the present disclosure.

Furthermore, although the numerative terminology, such as "first", "second", etc., may be used herein to describe various embodiments, elements or features, it should be understood that these embodiments, elements or features should not be limited by this numerative terminology. This numerative terminology is used herein only to distinguish one embodiment, element or feature from another embodiment, element or feature. For example, a first chamber discussed below could be called a second chamber, and vice versa, without departing from the teachings of the present disclosure.

In the embodiments disclosed herein, a cell may refer to a biological cell, such as a plant cell, an animal cell (e.g., a mammalian cell), a bacterial cell, a fungal cell, or the like. A mammalian cell may be, for example, from a human, a mouse, a horse, a goat, a sheep, a cow, a primate, or the like.

As used in the embodiments disclosed herein, a culture medium, also referred to as a growth medium, may refer to a liquid, suspension, or gel designed to support cellular growth in an artificial environment, i.e., *in vitro.* There are different types of culture media suitable for growing different types of cells. In general, the culture media may be broken into two primary categories: natural media and synthetic media. The natural media are those that are derived from tissue extraction or animal body fluids, such as plasma, lymph, and serum. The synthetic media are those created using a variety of organic and inorganic compounds. Moreover, the culture medium itself may comprise cells, bodies of cells (e.g., organoids), lipid particles, including natural or engineered (e.g., exosomal microvesicles, vacuoles, micelles or lipid particles of various design, virus particles, nanoparticles, etc.).

In the embodiments disclosed herein, a cell culture apparatus may refer to an apparatus having various wells (e.g., reservoirs, chambers, etc.) connected by microchannels in which fluids (i.e., culture media) will exhibit microfluidic behavior in their flow through the microchannels. Such a cell culture apparatus is also referred to as a microfluidic chip in this technical field. The microfluidic cell culture performed by the cell culture apparatus is generally related to cell culture, maintenance and perturbation in micro-scale fluid volumes. The reasons behind the popularity of the microfluidic cell culture are both economic and scientific. The microfluidic chips have the advantages of *in vitro* cell culture (high-throughput, parallel experiments, experiments may be done at the discretion of an experimenter, no need for specialized infrastructure and personnel, etc.) with *in vivo* like performance. For example, in mouse models, drugs are really selected for mice, not humans. By using human cells, drugs are screened for humans. Thus, using humanized microfluidic cell and tissue cultures to screen drug candidates reduces pre-clinical trial time and those drugs entering clinical testing are better suited for humans. This may reduce the probability of adverse effects and increase the chance of showing efficacy resulting in less failures in clinical trials.

In the embodiments disclosed herein, each microchannel of the cell culture apparatus is also referred to as a flow channel and may relate to a sub-millimeter-scale channel that has a hydraulic diameter below 1 mm and is used for fluidically connecting the wells of the cell culture apparatus. In other words, the microchannel or flow channel generally denotes a channel configured to pass different fluids, such, for example, as culture media (e.g., cell suspensions), reagents, or gels, in micro-scale volumes. The microchannel may be shaped such that it has appropriate flow characteristics (e.g., flow rates) depending on particular applications. For example, the microchannel may have a rectangular, e.g., square, or rounded cross-section, as well as be straight, bended, or tilted towards a required direction.

The microfluidic cell culture may involve using a porous membrane sandwiched between two flow layers within a microchannel or between a culture chamber and a microchannel for cell deposition. However, the membrane-based cell culture apparatuses known so far suffer from low throughput and incompatibility with available liquid handling systems (e.g., microtiter plate formats) and imaging systems. Furthermore, the flow control in such apparatuses is often limited to one method and not flexible enough to adapt to different resource settings. On top of that, the cell deposition on the basal (i.e., bottom) surface of the porous membrane(s) is possible in the current cell culture apparatuses only with very high cell concentrations, liquid flow control, and by inverting the cell culture apparatus.

The exemplary embodiments disclosed herein provide a technical solution that allows mitigating or even eliminating the drawbacks of the prior art. In particular, the technical solution disclosed herein provides a cell culture apparatus comprising a plurality of cell culture modules arranged adjacent to each other. Each cell culture module comprises two or more culture medium reservoirs connected by two or more flow channels. The flow channels go through a basal chamber arranged under an apical chamber. The apical and basal chambers are separated by a porous membrane. The basal chamber has a bottom part arranged higher than a bottom part of each of the culture medium reservoirs. In this apparatus configuration, flows of culture media may be perfused and regulated between the culture medium reservoirs in each cell culture module by either placing the apparatus on a rocking platform or connecting the apparatus to a pneumatic pump. Moreover, the apparatus thus configured may cultivate cells of the same or different types on the basal surface of the porous membranes in the cell culture modules under uniform and controlled *in vitro* conditions. The deposition of the cells on the basal surface of the porous membranes may be provided without having to invert the apparatus and to use high concentrations of the cells in the culture medium.

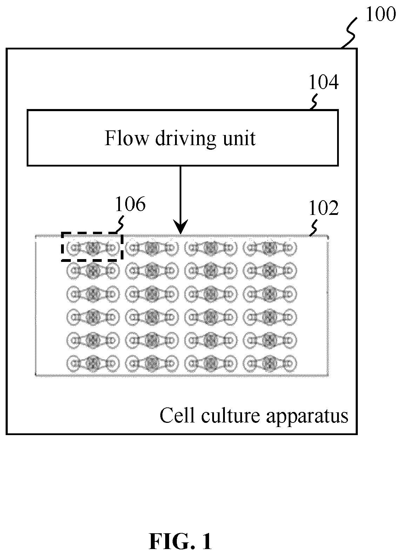

FIG. 1 shows a block diagram of a cell culture apparatus 100 in accordance with one exemplary embodiment. The apparatus 100 is intended for the above-mentioned microfluidic cell culture. As shown in FIG. 1, the apparatus 100 comprises a cell culture plate 102 and a flow driving unit 104 coupled to the cell culture plate 102. The cell culture plate 102 comprises multiple cell culture modules 106 and may be configured to fit the standard 96, 384, or 1536 microtiter plates. The flow driving unit 104 is configured to cause a culture medium to flow in each cell culture module 106, as will be described below in more detail. It should be noted that the number, arrangement, and interconnection of the constructive elements constituting the apparatus 100, which are shown in FIG. 1, are not intended to be any limitation of the present invention, but merely used to provide a general idea of how the constructive elements may be implemented within the apparatus 100. For example, the cell culture plate 102 may be replaced with two or more cell culture plates, and the flow driving unit 104 may be replaced with two or more flow driving units each configured to control a culture medium flow in one of the cell culture plates.

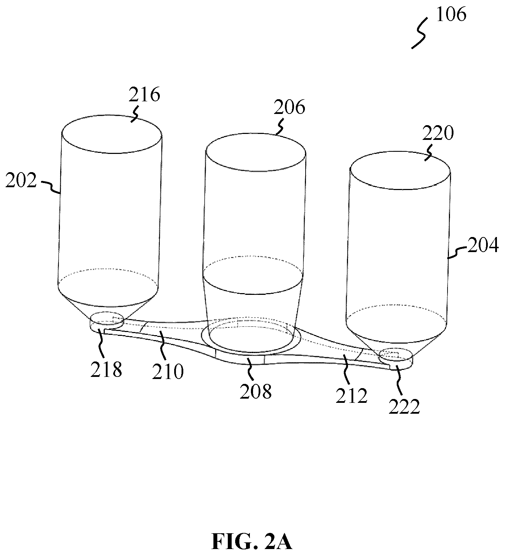

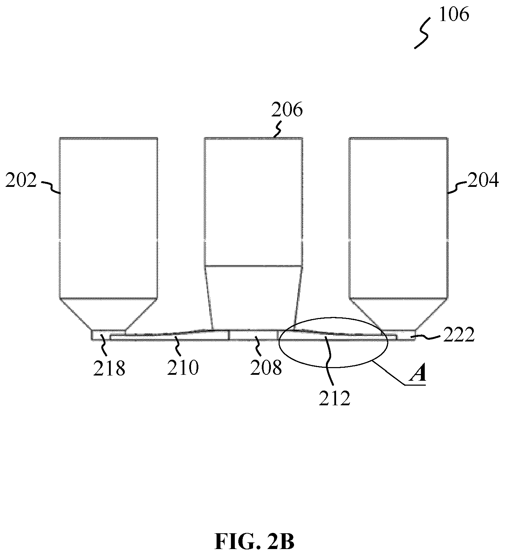

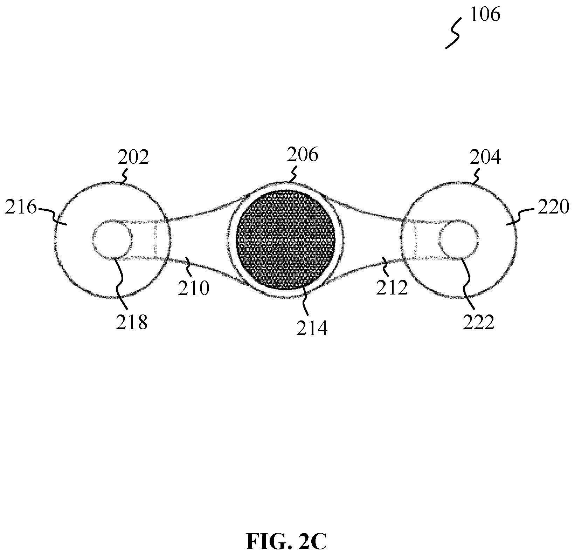

FIGs. 2A-2C show different schematic views of the cell culture module 106 included in the apparatus 100 in accordance with a first exemplary embodiment. More specifically, FIG. 2A shows a schematic perspective view of the cell culture module 106, FIG. 2B shows a schematic side view of the cell culture module 106, and FIG. 2C shows a schematic top view of the cell culture module 106. As shown in FIGs. 2A-2C, the cell culture module 106 comprises a first culture medium reservoir 202, a second culture medium reservoir 204, a first (apical or top) chamber 206, a second (basal or bottom) chamber 208, a first flow channel 210, a second flow channel 212, and a membrane 214. It should be again noted that the present disclosure is not limited to the shown number, arrangement, and interconnection of the constructive elements of the cell culture module 106. In some embodiments, there may be more than two medium culture reservoirs, thereby leading to more than two flow channels. In other embodiments, there may be more than two apical chambers and more than two basal chambers between the two culture reservoirs, thereby also increasing the number of the flow channels connecting the two culture reservoirs through the apical chambers and the basal chambers.

The first culture medium reservoir 202 has a top part with an inlet 216 for the culture medium and a bottom part with an outlet 218 for the culture medium. Similarly, the second culture medium reservoir 204 has a top part with an inlet 220 for the culture medium and a bottom part with an outlet 222 for the culture medium. The first and second culture medium reservoirs 202 and 204 may be implemented as identical hollow tubes. Although FIGs. 2A and 2C show that each of the first and second culture medium reservoirs 202 and 204 has a circular cross-section, this should not be construed as any limitation of the present disclosure; in some embodiments, any other cross-sectional shapes, such as polygonal, oval, etc., are possible, if required and depending on particular applications. Moreover, the bottom part of each of the first and second culture medium reservoirs 202 and 204 may have a different profile, for example, a positively tapered profile as shown in FIGs. 2A and 2B.

The first chamber 206 is arranged between the first and second medium culture reservoirs 202 and 204 such that the first chamber 206 and the first and second culture medium reservoirs 202 and 204 are aligned with each other in a first (horizontal) direction. The first chamber 206 may be implemented as a bottomless hollow tube, for example, with a positively tapered profile (as shown in FIGs. 2A and 2B) or with a uniform cross-section. Again, the shown circular cross-section of the first chamber 206 is for illustrative purposes only and should not be considered as any limitation of the present disclosure.

The second chamber 208 is arranged under the first chamber 206 and aligned with the first chamber 206 in a second (vertical) direction. The second chamber 208 may have a cross-section corresponding to the cross-section of the first chamber 206. The second chamber 208 has a bottom part having two or more lateral holes (not shown in FIGs. 2A-2C). As shown in FIGs. 2A-2C, the second chamber 208 has dimensions significantly smaller than the first chamber 206. For example, if the first chamber has a height of about 7 mm, then the height of the second chamber 208 may fall within a range from 50 µm to 150 µm (at height values higher than 150 µm, the second chamber 208 may lose microfluidic properties). Such height values of the first and second chambers 206 and 208 (in addition to the heights of the reservoirs 202 and 204) make the cell culture module 106 more compact, thereby reducing the total dimensions of the apparatus 100.

The membrane 214 has through pores (see FIG. 2C) and is arranged between the first chamber 206 and the second chamber 208 such that the first chamber 206 and the second chamber 208 are in flow communication with each other via the through pores of the membrane 214. The membrane 214 may be composed of silicone, plastic, protein, natural polymers, artificial polymers (e.g., polyester (PET)), metallic polymers or carbohydrates (e.g., cellulose), and may be formed by using one of the following methods: lithography, stamping, casting, electrospinning or *in situ* polymerization. The through pores may have different cross-sectional shapes, such, for example, as triangular, rectangular, square, oval, or polygonal (e.g., hexagonal). The polygonal cross-section shape of the through pores may refer to a convex or concave polygon. In some other embodiments, the through pores may have different cross-sectional shapes, such as any combination of two or more of the above-mentioned cross-sectional shapes (e.g., the combination of circular and square cross-section shapes). Furthermore, each of the through pores may have a pore size varying within a predefined range of values. For example, the pore size may vary from 0.2 µm to 10 µm. In general, the predefined range of values for the pore size (as well as the spacing between the through pores) is selected based on certain cells to be deposited on the membrane 214. More specially, the pore sizes may be more or less than sizes of the cells to be deposited on the membrane. For example, if the pore size is less than the cell size, then it is possible to prevent the cells from migrating between the basal and apical surfaces of the membrane 214.

The first and second flow channels 210 and 212 are microchannels providing the passage of the culture medium between the first and second culture medium reservoirs 202 and 204 through the second chamber 208. In particular, the first flow channel 210 connects the outlet 218 of the bottom part of the first culture medium reservoir 202 to one or more of the lateral holes arranged on the left side of the bottom part of the second chamber 208. The second flow channel 212 connects the outlet 222 of the bottom part of the second culture medium reservoir 204 to one or more of the lateral holes arranged on the right side of the bottom part of the second chamber 208. Each of the first and second flow channels 210 and 212 may have a longitudinal section and a cross-section which allow one to achieve required flow characteristics (e.g., an appropriate flow rate). For example, each of the first and second flow channels 210 and 212 may a variable channel height and a variable channel width which increase (e.g., gradually) towards the second chamber 208.

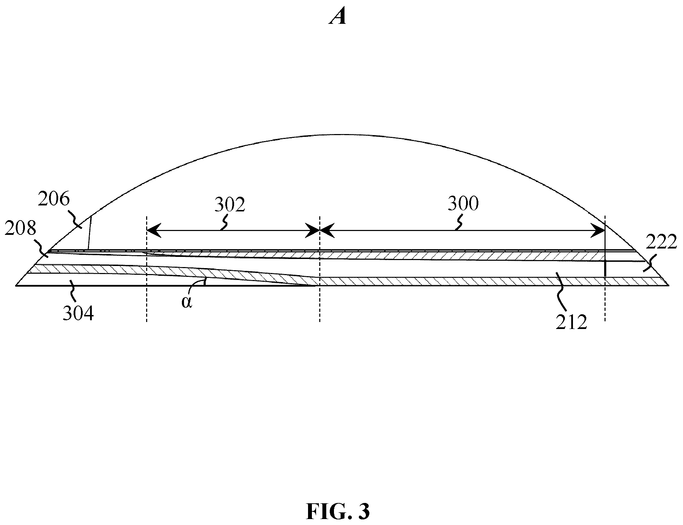

FIG. 3 shows a sectional view of the second flow channel 212 of the cell culture module 106, as taken within the area delimited by oval A in FIG. 2B. It should be noted that the first flow channel 210 has a similar sectional view (one just needs to mirror the shown sectional view). As shown in FIG. 3, the second flow channel 212 comprises a first channel portion 300 and a second channel portion 302. The first channel portion 300 extends from the outlet 222 of the bottom part of the second culture medium reservoir 204 and is parallel to the first (horizontal) direction. The second channel portion 302 connects the first channel portion 300 to the lateral hole(s) (not shown in FIG. 3) arranged on the right side of the second chamber 208. The second channel portion is tilted up (i.e., at a tilting angle α to the first direction) such that the bottom part of the second chamber 208 is arranged higher than the bottom part of the second culture medium reservoir 204 (i.e., higher than the end of the outlet 222). This tilt of the second channel portion 302 is provided by a small "hill" 304 which supports the second chamber 208. The tilting angle α of the second channel portion 302 relative to the first (horizontal) direction preferably falls within a range of 5 degrees to 45 degrees to provide appropriate flow characteristics. In one other embodiment, each of the first and second flow channels 210 and 212 may be fully tilted up at the tilting angle α, starting from the outlet of the corresponding culture medium reservoir (i.e., there may be no first channel portion parallel to the first (horizontal) direction).

In one embodiment, each of the second chamber 208, the first flow channel 210, and the second flow channel 212 may be coated, from inside, with a cell adhesion enhancing layer. The cell adhesion enhancing layer may be made of a hydrophilic material. Some non-restrictive examples of the hydrophilic material may include Matrigel, fibronectin, hyaluronic acid, different types of collagen (e.g., a type I collagen which is suitable for creating a blood-brain barrier cell culture model based on the apparatus 100), laminins, tenascin, elastane, nanocellulose with a host of different molecules. Such cell adhesion enhancing layers allow for less cells to be used in an experiment and more precise deposition of the cells on the basal surface of the membrane 214.

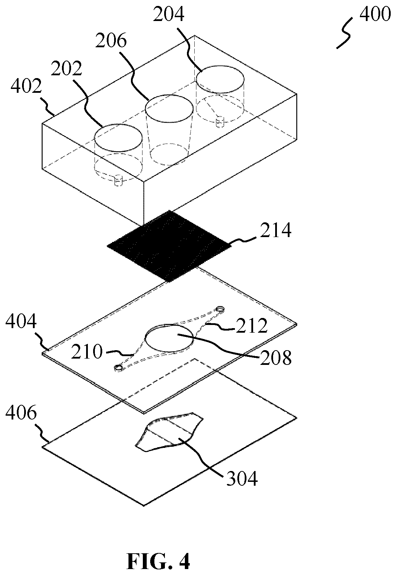

FIG. 4 shows a schematic exploded view of a layered structure 400 that represents one possible implementation example of the cell culture module 106. As shown in FIG. 4, the layered structure 400 comprises a first layer 402, a second layer 404, and a third layer 406. The first layer 402 comprises the first and second culture medium reservoirs 202 and 204, as well as the first chamber 206. The second layer 404 comprises the second chamber 208 arranged exactly under the membrane 214, as well as the flow channels 210 and 212. The membrane 214 is arranged between the first chamber 206 and the second chamber 208 to provide the fluid communication therebetween via the through pores. Moreover, the inlets of the flow channels 210 and 212 are arranged exactly under the outlets 218 and 222 (not shown in FIG. 4) of the culture medium reservoirs 202 and 204, respectively, to provide the fluid communication between the reservoirs 202 and 204 and the flow channels 210 and 212, respectively. The third layer 406 comprises the small "hill" 304 which is formed such that the appropriate tilting angle α of each of the flow channels 210 and 212 is provided. It should be noted that each of the first layer 402, the second layer 404, and the third layer 406 may be made of room-temperature-vulcanizing (RTV) silicone (e.g., RTV615) or polydimethylsiloxane (PDMS). Moreover, the layers 402-406 may be attached to each other in the order shown in FIG. 4 by using adhesive or plasma bonding. The cell culture modules 106 thus configured may be attached to each other by the using the same adhesive or plasma bonding to implement the cell culture plate 102 of the apparatus 100.

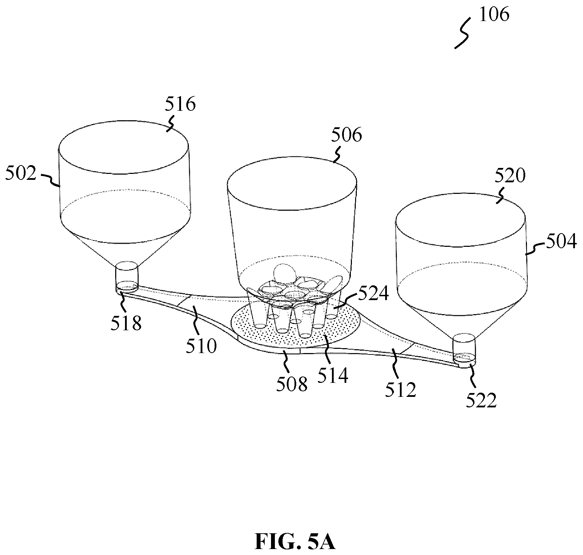

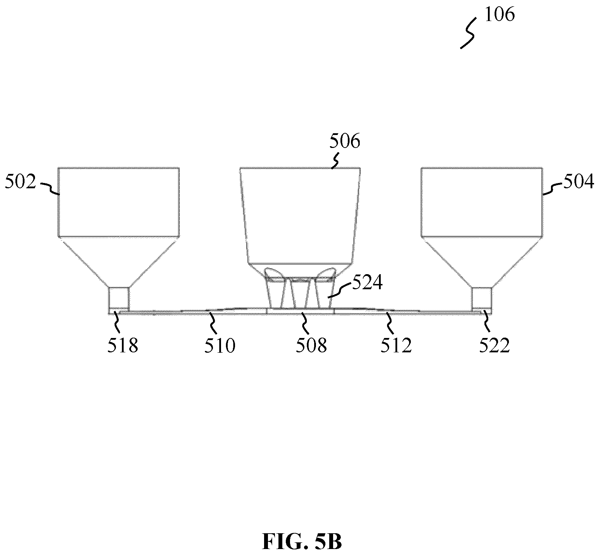

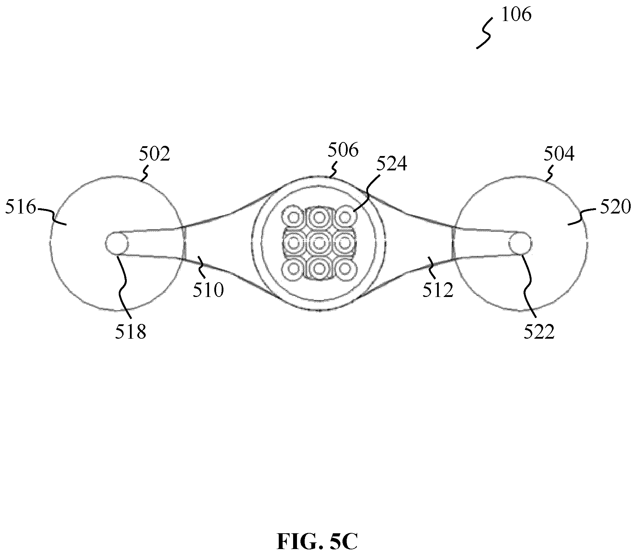

FIGs. 5A-5C show different schematic views of a cell culture module 106 included in the apparatus 100 in accordance with a second exemplary embodiment. More specifically, FIG. 5A shows a schematic perspective view of the cell culture module 106, FIG. 5B shows a schematic side view of the cell culture module 106, and FIG. 5C shows a schematic top view of the cell culture module 106. As shown in FIGs. 5A-5C, the cell culture module 106 comprises a first culture medium reservoir 502, a second culture medium reservoir 504, a first (apical or top) chamber 506, a second (basal or bottom) chamber 508, a first flow channel 510, a second flow channel 512, and a membrane 514. The first culture medium reservoir 502 has a top part with an inlet 516 for the culture medium and a bottom part with an outlet 518 for the culture medium. Similarly, the second culture medium reservoir 504 has a top part with an inlet 520 for the culture medium and a bottom part with an outlet 522 for the culture medium. It should be again noted that the present disclosure is not limited to the shown number, arrangement, and interconnection of the constructive elements of the cell culture module 106. In general, the first culture medium reservoir 502, the second culture medium reservoir 504, the second chamber 508, the first flow channel 510, the second flow channel 512, and the membrane 514 may be implemented in the same or similar manner as the first culture medium reservoir 202, the second culture medium reservoir 204, the second chamber 208, the first flow channel 210, the second flow channel 212, and the membrane 214, respectively.

At the same time, unlike the first chamber 206, the first chamber 506 may be implemented as a hollow tube having a curved or angled bottom. As shown in FIGs 5A-5C, the curved or angled bottom of the first chamber 506 comprises nine cavities 524 each provided with one or more holes, so that the first chamber 506 and the second chamber 508 are in fluid communication via the through pores of the membrane 514. It should be obvious to those skilled in the art that the shown number, shape, and arrangement of the cavities 524 are for illustrative purposes only and should not be construed as any limitation of the present disclosure. For example, there may be a single cavity 524 with a single hole. In general, the configuration of the cavity or cavities 524 depends on particular applications (e.g., a number of organoids and/spheroids to be formed on the apical surface of the membrane 514). In one embodiment, the curved or angled bottom of the first chamber 506 may be coated, from outside, with a cell adhesion enhancing layer such that the holes of the cavities 524 remains open. The cell adhesion enhancing layer may be the same as the one mentioned earlier in accordance with the first embodiment of the cell culture module 106.

Referring back to FIG. 1, the flow driving unit 104 is configured to cause the culture medium to flow, in each cell culture module 106 of the cell culture plate 102, between the first and second culture medium reservoirs 202 (502) and 204 (504). In one embodiment, the flow driving unit 104 may be a rocking platform that is configured to periodically rock the cell culture plate 102, thereby providing the culture medium flow. In another embodiment, the flow driving unit 104 may be a pneumatic pump that is configured to supply a compressed gas or a pressurized air to the inlet of each of the first and second culture medium reservoirs 202 (502) and 204 (504) in each cell culture module 106 of the cell culture plate 102, thereby causing the culture medium flow therein. In yet another embodiment, the flow driving unit 104 may be configured to cause the culture medium flow in each cell culture module 106 of the cell culture plate 102 by pipetting the culture medium from the first culture medium reservoir 202 (502) to the second culture medium reservoir 204 (504), or vice versa, at regular time intervals or based on a level of the culture medium in each of the reservoirs. Thus, unlike the current microfluidic cell culture devices, the apparatus 100 may be compatible with at least three different flow control methods which are indicated above.

In one embodiment, the apparatus 100 may further comprise a variety of electrodes. These electrodes may serve different purposes, and their arrangement inside the apparatus 100 depends on which purpose they are used for. In one embodiment, one or more cell culture modules of the cell culture plate 102 may comprise a first electrode arranged in the first chamber 206 (506) and a second electrode arranged in the second chamber 208 (508). In another embodiment, the first electrode may be arranged in the first culture medium reservoir 202 (502) and/or the second culture medium reservoir 204 (504) in one or more culture modules 106, while the second electrode may be arranged in the first chamber 206 (506) of the cell culture module(s) 106. Both these embodiments with the first and second electrodes may be, for example, used to perform real-time Transepithelial/Transendothelial Electrical Resistance (TEER) measurements. In this case, the first and second electrodes may be connected to an external control unit.

Additionally or alternatively, the apparatus 100 may further comprise two or more third electrodes arranged in the first chamber 206 (506) of one or more cell culture modules 106. These third electrodes may be arranged on the chamber walls for measuring a fluid level inside the first chamber 206 (506). In this case, the third electrodes may either be in direct contact with the fluid/culture medium to determine the fluid level by performing conductivity measurements, or be embedded into the chamber walls (e.g., at a distance of 0.5 mm - 2 mm from each other) to determine the fluid level by measuring inductive or capacitive changes in the third electrodes. At the same time, the two third electrodes may be arranged opposite to each other such that one of the two third electrodes is used to issue an ultrasound wave and another of the two third electrodes is used to receive the ultrasound wave passed through the interior of the first chamber 206 (506) (these ultrasound measurements may be performed just like radar measurements).

Additionally or alternatively, the apparatus 100 may further comprise two or more fourth electrodes embedded into the membrane 214 (514) of one or more cell culture modules 106. These fourth electrodes may be used to provide different stimulus signals to cells deposited on the apical and/or basal surface of the membrane.

In one embodiment, the first chamber 206 (506) or the second chamber 208 (508) of one or more cell culture modules 106 of the cell culture plate 102 may further comprise an oxygen sensor, a pH sensor, a CO₂ sensor, or any combination thereof. These sensors may be used to enable real-time measurements of different parameters crucial for cell behavior.

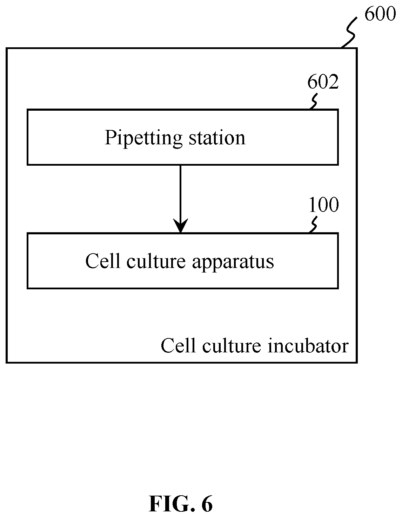

FIG. 6 shows a block diagram of a cell culture incubator 600 in accordance with one exemplary embodiment. The cell culture incubator 600 comprises the cell culture apparatus 100, and a pipetting station 602 coupled to the apparatus 100. More specifically, the pipetting station 602 is configured to feed the culture medium to each of the first and second culture medium reservoirs 202 (502) and 204 (504) in each cell culture module 106 of the cell culture plate 102. The pipetting station 602 is well-known in this technical field, for which reason its description is omitted herein.

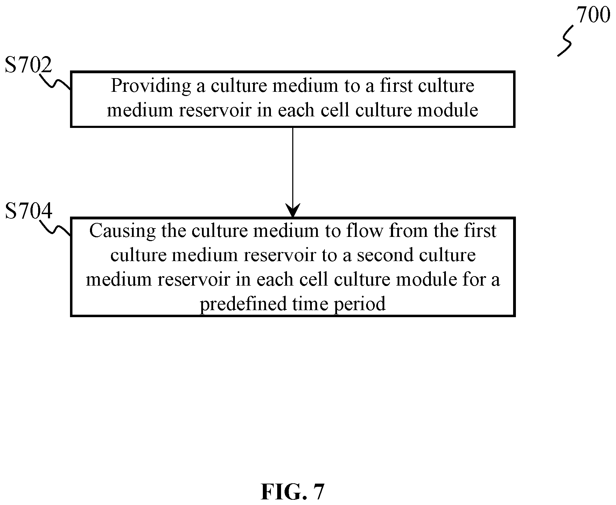

FIG. 7 shows a flowchart of a method 700 for cell cultivation by using the cell culture apparatus 100 in accordance with a first exemplary embodiment. The method 700 starts with a step S702, in which a required culture medium is provided, e.g., the pipetting station 602, to one of the first and second culture medium reservoirs 202 (502) and 204 (504) in each cell culture module 106 of the cell culture plate 102. Let us assume that the culture medium is provided to the first culture medium reservoir 202 (502). Then, the method 700 proceeds to a step S704, in which the flow driving unit 104 causes the culture medium to flow from the first culture medium reservoir 202 (502) to the second culture medium reservoir 204 (504) in each cell culture module 106 of the cell culture plate 102 for a predefined time period. The predefined time period may be selected based on the culture medium (i.e., a type of cells used therein). In particular, the cell deposition on the basal surface of the membrane 214 may be achieved if a flow rate is greater than gravity and a continuous flow lasts at least 30 min, preferably 60 min.

In one embodiment, the method 700 may comprise, during the step S704, an additional step, in which a different culture medium is fed, e.g., by the pipetting station 602, via the first chamber 206 (506) towards the membrane 214 (514) in one or more cell culture modules 106 of the cell culture plate 102. If the first chamber 206 is used, a cell monolayer is formed on the apical surface of the membrane 214, and this cell monolayer interacts with a cell layer forming on the basal surface of the membrane 214 via the through pores of the membrane 214. If the first chamber 506 is used, multiple microcavities 524 are bonded to the membrane 514, for which reason cells from the different culture medium are trapped to form a single organoid in each cavity. In this case, the flow channels 510 and 512 may perfuse the culture medium through the membrane 514 to keep the organoids alive.

In one embodiment, the step S704 of the method 700 may be performed by applying a positive pressure to the first and second culture medium reservoirs 210 (510) and 212 (512) in each cell culture module 106 of the cell culture plate 102 for the predefined time period (e.g., 60 min). In another embodiment, the step S704 of the method 700 may be performed by applying a positive pressure to the first two culture medium reservoir 202 (502 in each cell culture module 106 of the cell culture plate 102 for the predefined time period (e.g., 60 min), while capping the second culture medium reservoir 204 (504), or vice versa. In yet another embodiment, the step S704 of the method 700 may be performed by: (i) applying a first positive pressure to the first culture medium reservoir 202 (502) in each cell culture module 106 of the cell culture plate 102 for the predefined time interval, while capping the second culture medium reservoir 204 (504); and (ii) applying a second positive pressure to the second culture medium reservoir 204 (504) in each cell culture module 106 of the cell culture plate 102 for the predefined time interval, while capping the first culture medium reservoir 202 (502). The selection of each of the above-indicated embodiments of the step S704 depends on particular applications.

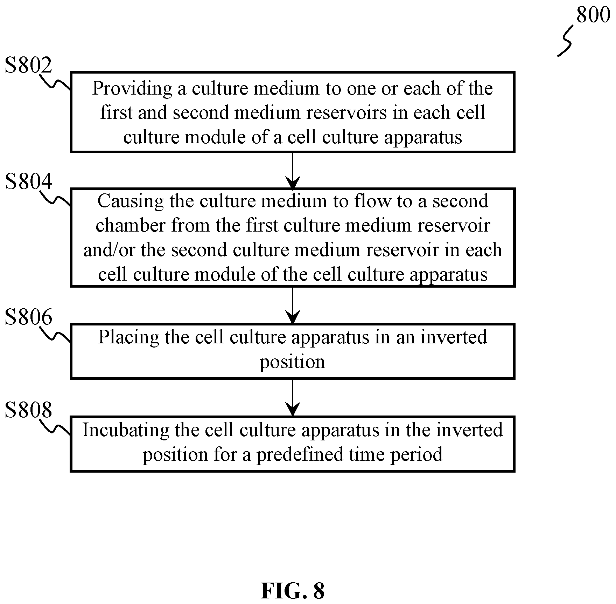

FIG. 8 shows a flowchart of a method 800 for cell cultivation by using the cell culture apparatus 100 in accordance with a second exemplary embodiment. The method 800 starts with a step S802, in which a required culture medium to one or each of the first and second medium reservoirs 202 (502) and 204 (504) in each cell culture module 106 of the cell culture plate 102. Then, the method 800 proceeds to a step S804, in which the flow driving unit 104 causes the culture medium to flow to the second chamber 208 (508) from the first medium culture reservoir 202 (502) and/or the second medium reservoir 204 (504) in each cell culture module 106 of the cell culture plate 102. After that, the method 800 goes on to a step S806, in which the cell culture plate 102 is placed in an inverted position. Next, the method 800 proceeds to a step S808, in which the cell culture plate 102 is incubated in the inverted position for a predefined time period. The predefined time period is again selected based on the culture medium.

In one embodiment, similar to the method 700, the method 800 further comprises, during the step S804, an additional step, in which a different culture medium is fed, e.g., by the pipetting station 602, via the first chamber 206 (506) towards the membrane 214 (514) in one or more cell culture modules 106 of the cell culture plate 102. If the first chamber 206 is used, a cell monolayer is formed on the apical surface of the membrane 214. If the first chamber 506 is used, a single organoid is formed in each cavity 524 of the first chamber 506. The culture medium perfused through the flow channels 210 (510) and 212 (512) may comprise human brain vascular endothelial cells, while the different culture medium fed to the first chamber 206 (505) may comprise human astrocytes. By using these types of cells, it is possible to mimic an artificial blood-brain barrier (BBB).

### Experimental results

The apparatus 100 comprising the cell culture modules like the one shown in FIGs. 2A-2C was used in accordance with the method 800 to obtain BBB models based on mouse astrocytes and human brain endothelial cells. Each BBB model was obtained in two stages which are described below in more detail.

### I. Stage of preparing the apparatus 100:

### Materials:

| | |
|---|---|
| Extracellular Matrix (ECM) coating solution (used as a cell adhesion enhancing layer for the flow channels 210, 212, the second chamber 208, and the first chamber 206) | Collagen I (Col-I) 100 µg/mL, 0.1% acetic acid |
| Washing | 1xPBS (phosphate buffer saline), ethanol 96% |
| Plate 102 | 96-well format |

The stage of preparing was performed as follows:
1. The dry apparatus 100 was treated with plasma for 60 s.
2. The apparatus 100 was washed with 96% ethanol. 50 µL of 96 % ethanol was added to each open-top (first) chamber 206.
3. The ethanol inside the flow channels 210, 212 and the first chamber 206 was replaced/washed twice with 50 µL of PBS.
4. 50 µL of Col-I coating solution was injected into the flow channels 210, 212.
5. 50 µL of Col-I coating solution was added into each first chamber 206 of the apparatus 100.
6. Each culture medium reservoir was filled with 50 µL PBS, closed, and incubated at 37°C, 5 % CO₂, for 1 hour on the rocking platform (1 cycle per 15 min).
7. After incubation, the coating solution in the flow channels 210, 212 and each first chamber 206 of the apparatus 100 was replaced with PBS.

### II. Stage of obtaining the BBB model:

### Materials:

| | |
|---|---|
| Cells used for the BBB model (concentration, volume) | Mouse astrocytes (MA) (2 × 10⁶ cells/mL, min. 4.8 mL), mouse primary brain microvascular endothelial cells (MPBMEC) (2 × 10⁶ cells/mL, min. 4.8 mL). |
| | Human astrocytes (HA) (2 × 10⁶ cells/mL, min. 4.8mL), human primary brain microvascular endothelial cells (HPBMEC) (2 × 10⁶ cells/mL, min. 4.8mL). |

The BBB model based on the mouse cells (i.e., MA-cells and MPBMEC) was obtained as follows:
1. 4800 µL MA-cells at 2 × 10⁶ cells/mL were prepared according to the conventional cell culture protocols.
2. The MA-cells were placed on ice.
3. The MA-cell loading was performed by:
   a. removing all the PBS from the first chambers 206,
   b. replacing the PBS in the flow channels 210, 212 with µL 100 endothelial cell medium,
   c. pipetting 50 µL MA-cell suspension at 2 × 10⁶ cells/mL into the first chambers 206,
   d. incubating the apparatus 100 at 37°C, 5 % CO₂, for 1 hour (static, not on the rocking platform),
   e. checking every 10 min that the membrane 214 does not dry out (if required, a fresh culture medium may be added to the first chamber(s) 206 to keep the membrane 214 moist).
4. 4800 µL MPBMEC at 2 × 10⁶ cells/mL were prepared according to the conventional cell culture protocols.
5. The MPBMEC were again placed on ice.
6. The cell culture medium (added in the apparatus in step 3e) was replaced by injecting 50 µL MPBMEC suspension at 2 × 10⁶ cells/mL into the flow channels 210, 212 of the plate 102.
7. The plate 102 was manually rocked a few times to make sure the cell confluency is consistent.
8. The plate 102 was inverted and incubated at 37°C, 5 % CO₂, for 1 hour (static, not on the rocking platform).
9. 100 µL MPBMEC were added to the culture medium reservoir at one side of each flow channel in the plate 102.
10. 15 µL MPBMEC were added to the cell culture reservoir at one side of each flow channel in the plate 102.
11. The plate 102 was placed on the rocking platform at 37°C, 5 % CO₂.

The BBB model based on the human cells (i.e., HA-cells and HPBMEC) may be obtained in the same manner (one just needs to replace "MA" and "MPBMEC" with "HA" and "HPBMEC", respectively, in steps 1-11 above).

Thus, the MAs were introduced in the first chamber 206 and grown a monolayer on the apical surface of the membrane 214, while the MPBMEC were perfused through the second chamber 208 and deposited on the basal side of the membrane 214.

Subsequently, the cells were treated immunohistochemically with antibodies against CD31, glial fibrillary acidic protein (GFAP), Actin and DAPI (which is a small molecule that binds to DNA showing the cell nuclei).

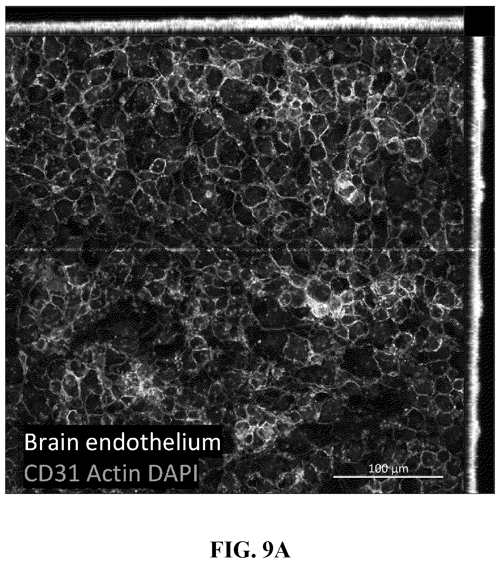

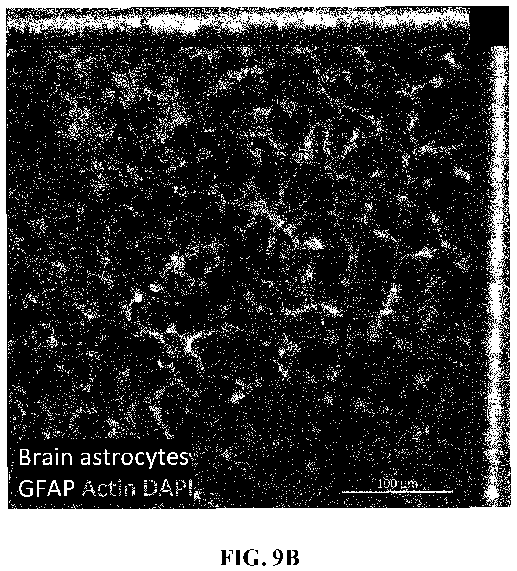

FIGs. 9A and 9B show confocal images of the BBB model by using the apparatus 100 in accordance with the method 800. More specifically, FIG. 9A shows the basal surface of the membrane 214, on which the MPBMEC were cultured. Adherens junction marker CD31 (used as the cell type-specific marker of the MPBMEC) and actin expression have shown robust expression and indicated intact cell junctions. FIG. 9B shows the apical surface of the membrane (from the side of the first chamber 206), on which the MAs were cultured. FIG. 9B shows the GFAP (used as the cell type-specific marker of the mouse brain astrocytes) and actin expression.

## Claims

1. A cell culture apparatus (100) comprising:
a plurality of cell culture modules (106) arranged adjacent to each other, each cell culture module (106) of the plurality of cell culture modules (106) comprising:
- at least two culture medium reservoirs (202, 204; 502, 504) each having a top part and a bottom part, the top part having an inlet (216, 220; 516, 520) for a culture medium and the bottom part having an outlet (218, 222; 518, 522) for the culture medium;
- a first chamber (206; 506) arranged between the at least two culture medium reservoirs (202, 204; 502, 504) such that the first chamber (206; 506) and the at least two culture medium reservoirs (202, 204; 502, 504) are aligned with each other in a first direction;
- a second chamber (208; 508) arranged under the first chamber (206; 506) and aligned with the first chamber (206; 506) in a second direction, the second direction being perpendicular to the first direction, the second chamber (208; 508) having a bottom part having at least two lateral holes, the bottom part of the second chamber (208; 508) being arranged higher than the bottom part of each of the at least two culture medium reservoirs (202, 204; 502, 504);
- a membrane (214; 514) having through pores formed therein, the membrane (214; 514) being arranged between the first chamber (206; 506) and the second chamber (208; 508) such that the first chamber (206; 506) and the second chamber (208; 508) are in flow communication with each other via the through pores; and
- at least two flow channels (210, 212; 510, 512) each connecting the outlet (218, 222; 518, 522) of the bottom part of one of the at least two culture medium reservoirs (202, 204; 502, 504) to one of the at least two lateral holes of the bottom part of the second chamber (208; 508); and
a flow driving unit (104) configured to cause the culture medium to flow, in each cell culture module (106) of the plurality of cell culture modules (106), between the at least two culture medium reservoirs (202, 204; 502, 504) via the at least two flow channels (210, 212; 510, 512) and the second chamber (208; 508).

2. The apparatus (100) of claim 1, wherein the first chamber (206) in at least one cell culture module (106) of the plurality of cell culture modules (106) is implemented as a bottomless hollow tube.

3. The apparatus (100) of claim 1, wherein the first chamber (506) in at least one cell culture module (106) of the plurality of cell culture modules (106) is implemented as a hollow tube having a curved or angled bottom, the curved or angled bottom having at least one cavity (524) and at least one hole formed in each of the at least one cavity (524).

4. The apparatus (100) of claim 3, wherein the curved or angled bottom is coated, from outside, with a cell adhesion enhancing layer such that the at least one hole of the at least one cavity (524) remains open, the cell adhesion enhancing layer being made of a hydrophilic material.

5. The apparatus (100) of any one of claims 1 to 4, wherein the second chamber (208; 508) has a height falling within a range from 50 µm to 150 µm.

6. The apparatus (100) of any one of claims 1 to 5, wherein the second chamber (208; 508) in at least one cell culture module (106) of the plurality of cell culture modules (106) is coated, from inside, with a cell adhesion enhancing layer, the cell adhesion enhancing layer being made of a hydrophilic material.

7. The apparatus (100) of any one of claims 1 to 6, wherein each of the at least two flow channels (210, 212; 510, 512) in at least one cell culture module (106) of the plurality of cell culture modules (106) is coated, from inside, with a cell adhesion enhancing layer, the cell adhesion enhancing layer being made of a hydrophilic material.

8. The apparatus (100) of any one of claims 1 to 7, wherein the flow driving unit (104) is:
- a pneumatic pump configured to supply a compressed gas or a pressurized air to the inlet (216, 220; 516, 520) of each of the at least two culture medium reservoirs (202, 204; 502, 504); or
- a pipetting means configured to pipette the culture medium from one of the at least two culture medium reservoirs (202, 204; 502, 504) to another of the at least two culture medium reservoirs (202, 204; 502, 504) at regular time intervals or based on a level of the culture medium in each of the at least two culture medium reservoirs (202, 204; 502, 504); or
- a rocking platform configured to periodically rock the plurality of cell culture modules (106).

9. The apparatus (100) of any one of claims 1 to 8, wherein each of the at least two culture medium reservoirs (202, 204; 502, 504) in at least one cell culture module (106) of the plurality of cell culture modules (106) is implemented as a hollow tube.

10. The apparatus (100) of any one of claims 1 to 9, wherein the bottom part of each of the at least two culture medium reservoirs (202, 204; 502, 504) has a positively tapered profile.

11. The apparatus (100) of any one of claims 1 to 10, wherein at least one cell culture module (106) of the plurality of cell culture modules (106) further comprises a first electrode arranged in the first chamber (206; 506) and a second electrode arranged in the second chamber (506; 508).

12. The apparatus (100) of any one of claims 1 to 10, wherein at least one cell culture module (106) of the plurality of cell culture modules (106) further comprises a first electrode arranged in one or more of the at least two culture medium reservoirs (202, 204; 502, 504) and a second electrode arranged in the first chamber (206; 506).

13. The apparatus (100) of any one of claims 1 to 12, wherein at least one cell culture module (106) of the plurality of cell culture modules (106) further comprises at least two third electrodes arranged in the first chamber (206; 506).

14. The apparatus (100) of any one of claims 1 to 13, wherein at least one cell culture module (106) of the plurality of cell culture modules (106) further comprises at least two fourth electrodes imbedded into the membrane (214; 514).

15. The apparatus (100) of any one of claims 1 to 14, wherein at least one cell culture module (106) of the plurality of cell culture modules (106) further comprises at least one of an oxygen sensor, a pH sensor and a CO₂ sensor in the first chamber (206; 506) or the second chamber (208; 508).

16. The apparatus (100) of any one of claims 1 to 15, wherein each of the at least two flow channels (210, 212; 510, 512) extends at least partly at a tilting angle (α) to the first direction, the tilting angle falling within a range of 5 degrees to 45 degrees.

17. The apparatus (100) of any one of claims 1 to 15, wherein the through pores of the membrane (214; 514) has an average pore size falling within a range from 0.2 µm to 10 µm.

18. The apparatus (100) of any one of claims 1 to 17, wherein each of the at least two flow channels (210, 212; 510, 512) in at least one cell culture module (106) of the plurality of cell culture modules (106) has a variable channel height and a variable channel width which increase towards the second chamber (208; 508).

19. The apparatus (100) of claim 18, wherein the variable channel width gradually increases towards the second chamber (208; 508).

20. The apparatus (100) of any one of claims 1 to 19, wherein each of the at least two flow channels (210, 212; 510, 512) in at least one cell culture module (106) of the plurality of cell culture modules (106) comprises a first channel portion (300) and a second channel portion (302), the first channel portion (300) extending from the outlet (218, 222; 518, 522) of the bottom part of one of the at least two culture medium reservoirs (202, 204; 502, 504) and being parallel to the first direction, the second channel portion (302) connecting the first channel portion (300) to one of the at least two lateral holes of the second chamber (208; 508), the second channel portion (302) being tilted relative to the first direction.

21. A cell culture incubator (600) comprising:
the cell culture apparatus (100) according to any one of claims 1 to 20; and
a pipetting station (602) configured to feed the culture medium to each of the at least two culture medium reservoirs (202, 204; 502, 504) in each cell culture module (106) of the plurality of cell culture modules (106).

22. A method (700) for cell cultivation by using the cell culture apparatus (100) according to any one of claims 1 to 20, the method (700) comprising:
(a) providing (S702) the culture medium to one of the at least two medium reservoirs (202, 204; 502, 504) in each cell culture module (106) of the plurality of cell culture modules (106); and
(b) causing (S704), by the flow driving unit (104), the culture medium to flow from said one of the at least two culture medium reservoirs (202, 204; 502, 504) to the rest of the at least two culture medium reservoirs (202, 204; 502, 504) in each cell culture module (106) of the plurality of cell culture modules (106) for a predefined time period, the predefined time period being selected based on the culture medium.

23. The method (700) of claim 22, further comprising, during step (b), feeding another culture medium via the first chamber (206; 506) towards the membrane (214; 514) in at least one cell culture module (106) of the plurality of cell culture modules (106).

24. The method (700) of claim 22 or 23, wherein step (b) comprises applying a positive pressure to the at least two culture medium reservoirs (202, 204; 502, 504) in each cell culture module (106) of the plurality of cell culture modules (106) for the predefined time period.

25. The method (700) of claim 22 or 23, wherein step (b) comprises applying a positive pressure to said one of the at least two culture medium reservoirs (202, 204; 502, 504) in each cell culture module (106) of the plurality of cell culture modules (106) for the predefined time period, while capping the rest of the at least two culture medium reservoirs (202, 204; 502, 504).

26. The method (700) of claim 22 or 23, wherein the at least two culture medium reservoirs (202, 204; 502, 504) in each cell culture module (106) of the plurality of cell culture modules (106) comprises a first culture medium reservoir (202; 502) and a second culture medium reservoir (204; 504), and wherein step (b) comprises:
- applying a first positive pressure to the first culture medium reservoir (202; 502) in each cell culture module (106) of the plurality of cell culture modules (106) for the predefined time interval, while capping the second culture medium reservoir (204; 504); and
- applying a second positive pressure to the second culture medium reservoir (204; 504) in each cell culture module (106) of the plurality of cell culture modules (106) for the predefined time interval, while capping the first culture medium reservoir (202; 502).

27. A method (800) for cell cultivation by using the cell culture apparatus (100) according to any one of claims 1 to 20, the method (800) comprising:
(a) providing (S802) the culture medium to one or more of the at least two medium reservoirs (202, 204; 502, 504) in each cell culture module (106) of the plurality of cell culture modules (106);
(b) causing (S804), by the flow driving unit (104), the culture medium to flow to the second chamber (208; 508) from said one or more of the at least two medium reservoirs (202, 204; 502, 504) in each cell culture module (106) of the plurality of cell culture modules (106);
(c) placing (S806) the cell culture apparatus (100) in an inverted position; and
(d) incubating (S808) the cell culture apparatus (100) in the inverted position for a predefined time period, the predefined time period being selected based on the culture medium.

28. The method (800) of claim 27, further comprising, during step (b), feeding another culture medium via the first chamber (206; 506) towards the membrane (214; 514) in at least one cell culture module (106) of the plurality of cell culture modules (106).

29. The method (800) of claim 28, wherein the culture medium comprises human brain vascular endothelial cells and said another culture medium comprises human astrocytes.

## Patentansprüche

1. Zellkulturgerät (100), umfassend:
eine Mehrzahl von Zellkulturmodulen (106), die benachbart zueinander angeordnet sind, wobei jedes Zellkulturmodul (106) von der Mehrzahl von Zellkulturmodulen (106) umfasst:
- wenigstens zwei Kulturmediumreservoirs (202, 204; 502, 504), die jeweils einen oberen Teil und einen Bodenteil haben, wobei der obere Teil einen Einlass (216, 220; 516, 520) für ein Kulturmedium hat, und der Bodenteil einen Auslass (218, 222; 518, 522) für das Kulturmedium hat;
- eine erste Kammer (206; 506), die zwischen den wenigstens zwei Kulturmediumreservoirs (202, 204; 502, 504) derart angeordnet ist, dass die erste Kammer (206; 506) und die wenigstens zwei Kulturmediumreservoirs (202, 204; 502, 504) miteinander in einer ersten Richtung ausgerichtet sind;
- eine zweite Kammer (208; 508), die unter der ersten Kammer (206; 506) angeordnet und mit der ersten Kammer (206; 506) in einer zweiten Richtung ausgerichtet ist, wobei die zweite Richtung orthogonal zu der ersten Richtung ist, wobei die zweite Kammer (208; 508) einen Bodenteil mit wenigstens zwei lateralen Löchern hat, wobei der Bodenteil der zweiten Kammer (208; 508) höher als der Bodenteil von jedem von den wenigstens zwei Kulturmediumreservoirs (202, 204; 502, 504) angeordnet ist;
- eine Membran (214; 514) mit darin gebildeten Durchgangsporen, wobei die Membran (214; 514) zwischen der ersten Kammer (206; 506) und der zweiten Kammer (208; 508) derart angeordnet ist, dass die erste Kammer (206; 506) und die zweite Kammer (208; 508) in Strömungsverbindung miteinander über die Durchgangsporen sind; und
- wenigstens zwei Strömungskanäle (210, 212; 510, 512), die jeweils den Auslass (218, 222; 518, 522) des Bodenteils von einem von den wenigstens zwei Kulturmediumreservoirs (202, 204; 502, 504) mit einem von den wenigstens zwei lateralen Löchern des Bodenteils der zweiten Kammer (208; 508) verbinden; und
eine Strömungsantriebseinheit (104), die dazu konfiguriert ist, das Strömen des Kulturmediums in jedem Zellkulturmodul (106) von der Mehrzahl von Zellkulturmodulen (106) zwischen den wenigstens zwei Kulturmediumreservoirs (202, 204; 502, 504) über die wenigstens zwei Strömungskanäle (210, 212; 510, 512) und die zweite Kammer (208; 508) zu bewirken.

2. Gerät (100) nach Anspruch 1, wobei die erste Kammer (206) in wenigstens einem Zellkulturmodul (106) von der Mehrzahl von Zellkulturmodulen (106) als ein bodenloses Hohlrohr implementiert ist.

3. Gerät (100) nach Anspruch 1, wobei die erste Kammer (506) in wenigstens einem Zellkulturmodul (106) von der Mehrzahl von Zellkulturmodulen (106) als ein Hohlrohr mit einem gekrümmten oder gewinkelten Boden implementiert ist, wobei der gekrümmte oder gewinkelte Boden wenigstens einen Hohlraum (524) und wenigstens ein Loch hat, das in jedem von dem wenigstens einen Hohlraum (524) gebildet ist.

4. Gerät (100) nach Anspruch 3, wobei der gekrümmte oder gewinkelte Boden von außen mit einer Zelladhäsionsverstärkungsschicht derart beschichtet ist, dass das wenigstens eine Loch des wenigstens einen Hohlraums (524) offen bleibt, wobei die Zelladhäsionsverstärkungsschicht aus einem hydrophilen Material gebildet ist.

5. Gerät (100) nach einem der Ansprüche 1 bis 4, wobei die zweite Kammer (208; 508) eine Höhe hat, die innerhalb eines Bereichs von 50µm bis 150µm fällt.

6. Gerät (100) nach einem der Ansprüche 1 bis 5, wobei die zweite Kammer (208; 508) in wenigstens einem Zellkulturmodul (106) von der Mehrzahl von Zellkulturmodulen von innen mit einer Zelladhäsionsverstärkungsschicht beschichtet ist, wobei die Zelladhäsionsverstärkungsschicht aus einem hydrophilen Material gebildet ist.

7. Gerät (100) nach einem der Ansprüche 1 bis 6, wobei jeder von den wenigstens zwei Strömungskanälen (210, 212; 510, 512) in wenigstens einem Zellkulturmodul (106) von der Mehrzahl von Zellkulturmodulen (106) von innen mit einer Zelladhäsionsverstärkungsschicht beschichtet ist, wobei die Zelladhäsionsverstärkungsschicht aus einem hydrophilen Material gebildet ist.

8. Gerät (100) nach einem der Ansprüche 1 bis 7, wobei die Strömungsantriebseinheit (104) Folgendes ist:
- eine pneumatische Pumpe, die dazu konfiguriert ist, ein komprimiertes Gas oder Druckluft zu dem Einlass (216, 220; 516, 520) von jedem von der wenigstens zwei Kulturmediumreservoirs (202, 204; 502, 504) zuzuführen; oder
- ein Pipettiermittel, das dazu konfiguriert ist, das Kulturmedium von einem der wenigstens zwei Kulturmediumreservoirs (202, 204; 502, 504) zu einem anderen der wenigstens zwei Kulturmediumreservoirs (202, 204; 502, 504) in regelmäßigen Zeitintervallen oder basierend auf einem Pegel des Kulturmediums in jedem von den wenigstens zwei Kulturmediumreservoirs (202, 204; 502, 504) zu pipettieren; oder
- eine Rüttelplattform, die dazu konfiguriert ist, die Mehrzahl von Zellkulturmodulen (106) periodisch zu rütteln.

9. Gerät (100) nach einem der Ansprüche 1 bis 8, wobei jedes von den wenigstens zwei Kulturmediumreservoirs (202, 204; 502, 504) in wenigstens einem Zellkulturmodul (106) von der Mehrzahl von Zellkulturmodulen (106) als ein Hohlrohr implementiert ist.

10. Gerät (100) nach einem der Ansprüche 1 bis 9, wobei der Bodenteil von jedem von den wenigstens zwei Kulturmediumreservoirs (202, 204; 502, 504) ein positives Verjüngungsprofil hat.

11. Gerät (100) nach einem der Ansprüche 1 bis 10, wobei wenigstens ein Zellkulturmodul (106) von der Mehrzahl von Zellkulturmodulen (106) ferner eine erste Elektrode umfasst, die in der ersten Kammer (206; 506) angeordnet ist, und eine zweite Elektrode, die in der zweiten Kammer (506; 508) angeordnet ist.

12. Gerät (100) nach einem der Ansprüche 1 bis 10, wobei wenigstens ein Zellkulturmodul (106) von der Mehrzahl von Zellkulturmodulen (106) ferner eine erste Elektrode umfasst, die in einem oder mehr von den wenigstens zwei Kulturmediumreservoirs (202, 204; 502, 504) angeordnet ist, und eine zweite Elektrode, die in der ersten Kammer (206; 506) angeordnet ist.

13. Gerät (100) nach einem der Ansprüche 1 bis 12, wobei wenigstens ein Zellkulturmodul (106) von der Mehrzahl von Zellkulturmodulen (106) ferner wenigstens zwei dritte Elektroden umfasst, die in der ersten Kammer (206; 506) angeordnet sind.

14. Gerät (100) nach einem der Ansprüche 1 bis 13, wobei wenigstens ein Zellkulturmodul (106) von der Mehrzahl von Zellkulturmodulen (106) ferner wenigstens zwei vierte Elektroden umfasst, die in die Membran (214; 514) eingebettet sind.

15. Gerät (100) nach einem der Ansprüche 1 bis 14, wobei wenigstens ein Zellkulturmodul (106) von der Mehrzahl von Zellkulturmodulen (106) ferner einen Sauerstoffsensor, eine pH-Sensor und/oder einen CO₂-Sensor in der ersten Kammer (206; 506) oder der zweiten Kammer (208; 508) umfasst.

16. Gerät (100) nach einem der Ansprüche 1 bis 15, wobei sich jeder von den wenigstens zwei Strömungskanälen (210, 212; 510, 512) wenigstens teilweise unter einem Neigungswinkel (α) zu der ersten Richtung erstreckt, wobei der Neigungswinkel innerhalb eines Bereichs von 5 Grad bis 45 Grad fällt.

17. Gerät (100) nach einem der Ansprüche 1 bis 15, wobei die Durchgangsporen der Membran (214; 514) eine durchschnittliche Porengröße haben, die innerhalb eines Bereichs von 0,2 µm bis 10 µm fällt.

18. Gerät (100) nach einem der Ansprüche 1 bis 17, wobei jeder von den wenigstens zwei Strömungskanälen (210, 212; 510, 512) in wenigstens einem Zellkulturmodul (106) von der Mehrzahl von Zellkulturmodulen (106) eine variable Kanalhöhe und eine variable Kanalbreite hat, die in Richtung der zweiten Kammer (208; 508) zunehmen.

19. Gerät (100) nach Anspruch 18, wobei die variable Kanalbreite schrittweise in Richtung der zweiten Kammer (208; 508) zunimmt.

20. Gerät (100) nach einem der Ansprüche 1 bis 19, wobei jeder von den wenigstens zwei Strömungskanälen (210, 212; 510, 512) in wenigstens einem Zellkulturmodul (106) von der Mehrzahl von Zellkulturmodulen (106) einen ersten Kanalbereich (300) und einen zweiten Kanalbereich (302) umfasst, wobei sich der erste Kanalbereich (300) von dem Auslass (218, 222; 518, 522) des Bodenteils von einem von den wenigstens zwei Kulturmediumreservoirs (202, 204; 502, 504) erstreckt und parallel zu der ersten Richtung ist, wobei der zweite Kanalbereich (302) den ersten Kanalbereich (300) mit einem von den wenigstens zwei lateralen Löchern der zweiten Kammer (208; 508) verbindet, wobei der zweite Kanalbereich (302) relativ zu der ersten Richtung geneigt ist.

21. Zellkulturinkubator (600), umfassend:
das Zellkulturgerät (100) nach einem der Ansprüche 1 bis 20; und eine Pipettierstation (602), die dazu konfiguriert ist, das Kulturmedium zu jedem von den wenigstens zwei Kulturmediumreservoirs (202, 204; 502, 504) in jedem Zellkulturmodul (106) von der Mehrzahl von Zellkulturmodulen (106) zuzuführen.

22. Verfahren (700) zur Zellkultivierung unter Verwendung des Zellkulturgeräts (100) nach einem der Ansprüche 1 bis 20, wobei das Verfahren (700) umfasst:
(a) Bereitstellen (S702) des Kulturmediums zu einem von den wenigstens zwei Mediumreservoirs (202, 204; 502, 504) in jedem Zellkulturmodul (106) von der Mehrzahl von Zellkulturmodulen (106); und
(b) Veranlassen (S704), durch die Strömungsantriebseinheit (104), dass das Kulturmedium von dem einen von den wenigstens zwei Kulturmediumreservoirs (202, 204; 502, 504) zu dem Rest der wenigstens zwei Kulturmediumreservoirs (202, 204; 502, 504) in jedem Zellkulturmodul (106) von der Mehrzahl von Zellkulturmodulen (106) für eine vordefinierte Zeitperiode strömt, wobei die vordefinierte Zeitperiode basierend auf dem Kulturmedium ausgewählt wird.

23. Verfahren (700) nach Anspruch 22, ferner umfassend während des Schritts (b) ein Zuführen eines anderen Kulturmediums über die erste Kammer (206; 506) in Richtung der Membran (214; 514) in wenigstens einem Zellkulturmodul (106) von der Mehrzahl von Zellkulturmodulen (106).

24. Verfahren (700) nach Anspruch 22 oder 23, wobei der Schritt (b) das Ausüben eines Überdrucks auf die wenigstens zwei Kulturmediumreservoirs (202, 204; 502, 504) in jedem Zellkulturmodul (106) von der Mehrzahl von Zellkulturmodulen (106) für die vordefinierte Zeitperiode umfasst.

25. Verfahren (700) nach Anspruch 22 oder 23, wobei der Schritt (b) das Ausüben eines Überdrucks auf das eine von den wenigstens zwei Kulturmediumreservoirs (202, 204; 502, 504) in jedem Zellkulturmodul (106) von der Mehrzahl von Zellkulturmodulen (106) für die vordefinierte Zeitperiode umfasst, während der Rest der wenigstens zwei Kulturmediumreservoirs (202, 204; 502, 504) geschlossen wird.

26. Verfahren (700) nach Anspruch 22 oder 23, wobei die wenigstens zwei Kulturmediumreservoirs (202, 204; 502, 504) in jedem Zellkulturmodul (106) von der Mehrzahl von Zellkulturmodulen (106) ein erstes Kulturmediumreservoir (202; 502) und ein zweites Kulturmediumreservoir (204; 504) umfasst, und wobei der Schritt (b) umfasst:
- Ausüben eines ersten Überdrucks auf das erste Kulturmediumreservoir (202; 502) in jedem Zellkulturmodul (106) von der Mehrzahl von Zellkulturmodulen (106) für das vordefinierte Zeitintervall, während das zweite Kulturmediumreservoir (204; 504) geschlossen wird; und
- Ausüben eines zweiten Überdrucks auf das zweite Kulturmediumreservoir (204; 504) in jedem Zellkulturmodul (106) von der Mehrzahl von Zellkulturmodulen (106) für das vordefinierte Zeitintervall, während das erste Kulturmediumreservoir (202; 502) geschlossen wird.

27. Verfahren (800) zur Zellkultivierung unter Verwendung des Zellkulturgeräts (100) nach einem der Ansprüche 1 bis 20, wobei das Verfahren (800) umfasst:
(a) Bereitstellen (S802) des Kulturmediums zu einem oder mehr von den wenigstens zwei Mediumreservoirs (202, 204; 502, 504) in jedem Zellkulturmodul (106) von der Mehrzahl von Zellkulturmodulen (106);
(b) Veranlassen (S804), durch die Strömungsantriebseinheit (104), dass das Kulturmedium zu der zweiten Kammer (208; 508) von dem einen oder mehr von den wenigstens zwei Mediumreservoirs (202, 204; 502, 504) in jedem Zellkulturmodul (106) von der Mehrzahl von Zellkulturmodulen (106) strömt;
(c) Platzieren (S806) des Zellkulturgeräts (100) in einer invertierten Position; und
(d) Inkubieren (S808) des Zellkulturgeräts (100) in der invertierten Position für eine vordefinierte Zeitperiode, wobei die vordefinierte Zeitperiode basierend auf dem Kulturmedium ausgewählt wird.

28. Verfahren (800) nach Anspruch 27, ferner umfassend, während des Schritts (b), das Zuführen eines anderen Kulturmediums über die erste Kammer (206; 506) in Richtung der Membran (214; 514) in wenigstens einem Zellkulturmodul (106) von der Mehrzahl von Zellkulturmodulen (106).

29. Verfahren (800) nach Anspruch 28, wobei das Kulturmedium menschliche Gehirngefäßendothelzellen umfasst, und das andere Kulturmedium menschliche Astrozyten umfasst.

## Revendications

1. Appareil (100) de culture cellulaire comprenant :
une pluralité de modules (106) de culture cellulaire agencés adjacents les uns aux autres, chaque module (106) de culture cellulaire de la pluralité de modules (106) de culture cellulaire comprenant :
- au moins deux réservoirs (202, 204 ; 502, 504) de milieu de culture présentant chacun une partie supérieure et une partie inférieure, la partie supérieure présentant une entrée (216, 220 ; 516, 520) pour un milieu de culture et la partie inférieure présentant une sortie (218, 222 ; 518, 522) pour le milieu de culture ;
- une première chambre (206 ; 506) agencée entre les au moins deux réservoirs (202, 204 ; 502, 504) de milieu de culture de telle sorte que la première chambre (206 ; 506) et les au moins deux réservoirs (202, 204 ; 502, 504) de milieu de culture sont alignés l'un avec l'autre dans une première direction ;
- une seconde chambre (208 ; 508) agencée sous la première chambre (206 ; 506) et alignée avec la première chambre (206 ; 506) dans une seconde direction,
la seconde direction étant perpendiculaire à la première direction, la seconde chambre (208 ; 508) présentant une partie inférieure présentant au moins deux trous latéraux, la partie inférieure de la seconde chambre (208 ; 508) étant agencée plus haut que la partie inférieure de chacun des au moins deux réservoirs (202, 204 ; 502, 504) de milieu de culture ;
- une membrane (214 ; 514) présentant des pores traversants formés dans celle-ci, la membrane (214 ; 514) étant agencée entre la première chambre (206 ; 506) et la seconde chambre (208 ; 508) de telle sorte que la première chambre (206 ; 506) et la seconde chambre (208 ; 508) sont en communication d'écoulement l'une avec l'autre via les pores traversants ; et
- au moins deux canaux d'écoulement (210, 212 ; 510, 512) reliant chacun la sortie (218, 222 ; 518, 522) de la partie inférieure de l'un des au moins deux réservoirs (202, 204 ; 502, 504) de milieu de culture à l'un des au moins deux trous latéraux de la partie inférieure de la seconde chambre (208 ; 508) ; et
une unité d'entraînement en écoulement (104) configurée pour amener le milieu de culture à s'écouler, dans chaque module (106) de culture cellulaire de la pluralité de modules (106) de culture cellulaire, entre les au moins deux réservoirs (202, 204 ; 502, 504) de milieu de culture via les au moins deux canaux d'écoulement (210, 212 ; 510, 512) et de la seconde chambre (208 ; 508).

2. Appareil (100) selon la revendication 1, dans lequel la première chambre (206) dans au moins un module (106) de culture cellulaire de la pluralité de modules (106) de culture cellulaire est mise en œuvre sous la forme d'un tube creux sans fond.

3. Appareil (100) selon la revendication 1, dans lequel la première chambre (506) dans au moins un module (106) de culture cellulaire de la pluralité de modules (106) de culture cellulaire est mise en œuvre sous la forme d'un tube creux présentant un fond incurvé ou incliné, le fond incurvé ou incliné présentant au moins une cavité (524) et au moins un trou formé dans chacune de la au moins une cavité (524).

4. Appareil (100) selon la revendication 3, dans lequel le fond incurvé ou incliné est recouvert, de l'extérieur, d'une couche d'amélioration d'adhérence cellulaire de telle sorte que le au moins un trou de la au moins une cavité (524) reste ouvert, la couche d'amélioration d'adhérence cellulaire étant composée d'un matériau hydrophile.

5. Appareil (100) selon l'une quelconque des revendications 1 à 4, dans lequel la seconde chambre (208 ; 508) présente une hauteur tombant dans une plage de 50 µm à 150 µm.

6. Appareil (100) selon l'une quelconque des revendications 1 à 5, dans lequel la seconde chambre (208 ; 508) dans au moins un module (106) de culture cellulaire de la pluralité de modules (106) de culture cellulaire est recouverte, de l'intérieur, d'une couche d'amélioration d'adhérence cellulaire, la couche d'amélioration d'adhérence cellulaire étant composée d'un matériau hydrophile.

7. Appareil (100) selon l'une quelconque des revendications 1 à 6, dans lequel chacun des au moins deux canaux d'écoulement (210, 212 ; 510, 512) dans au moins un module (106) de culture cellulaire de la pluralité de modules (106) de culture cellulaire est recouvert, de l'intérieur, d'une couche d'amélioration d'adhérence cellulaire, la couche d'amélioration d'adhérence cellulaire étant composée d'un matériau hydrophile.

8. Appareil (100) selon l'une quelconque des revendications 1 à 7, dans lequel l'unité d'entraînement en écoulement (104) est :
- une pompe pneumatique configurée pour apporter un gaz comprimé ou un air sous pression à l'entrée (216, 220 ; 516, 520) de chacun des au moins deux réservoirs (202, 204 ; 502, 504) de milieu de culture ; ou
- un moyen de pipetage configuré pour pipeter le milieu de culture depuis l'un des au moins deux réservoirs (202, 204 ; 502, 504) de milieu de culture vers un autre des au moins deux réservoirs (202, 204 ; 502, 504) de milieu de culture à des intervalles de temps réguliers ou sur la base d'un niveau du milieu de culture dans chacun des au moins deux réservoirs (202, 204 ; 502, 504) de milieu de culture ; ou - une plateforme basculante configurée pour faire basculer périodiquement la pluralité de modules (106) de culture cellulaire.

9. Appareil (100) selon l'une quelconque des revendications 1 à 8, dans lequel chacun des au moins deux réservoirs (202, 204 ; 502, 504) de milieu de culture dans au moins un module (106) de culture cellulaire de la pluralité de modules (106) de culture cellulaire est mis en œuvre sous la forme d'un tube creux.

10. Appareil (100) selon l'une quelconque des revendications 1 à 9, dans lequel la partie inférieure de chacun des au moins deux réservoirs (202, 204 ; 502, 504) de milieu de culture présente un profil conique positif.

11. Appareil (100) selon l'une quelconque des revendications 1 à 10, dans lequel au moins un module (106) de culture cellulaire de la pluralité de modules (106) de culture cellulaire comprend en outre une première électrode agencée dans la première chambre (206 ; 506) et une deuxième électrode agencée dans la seconde chambre (506 ; 508).

12. Appareil (100) selon l'une quelconque des revendications 1 à 10, dans lequel au moins un module (106) de culture cellulaire de la pluralité de modules (106) de culture cellulaire comprend en outre une première électrode agencée dans un ou plusieurs des au moins deux réservoirs (202, 204 ; 502, 504) de milieu de culture et une deuxième électrode agencée dans la première chambre (206 ; 506).

13. Appareil (100) selon l'une quelconque des revendications 1 à 12, dans lequel au moins un module (106) de culture cellulaire de la pluralité de modules (106) de culture cellulaire comprend en outre au moins deux troisièmes électrodes agencées dans la première chambre (206 ; 506).

14. Appareil (100) selon l'une quelconque des revendications 1 à 13, dans lequel au moins un module (106) de culture cellulaire de la pluralité de modules (106) de culture cellulaire comprend en outre au moins deux quatrièmes électrodes incorporées dans la membrane (214 ; 514).

15. Appareil (100) selon l'une quelconque des revendications 1 à 14, dans lequel au moins un module (106) de culture cellulaire de la pluralité de modules (106) de culture cellulaire comprend en outre au moins un parmi un capteur d'oxygène, un capteur de pH et un capteur de CO₂ dans la première chambre (206 ; 506) ou la seconde chambre (208 ; 508).

16. Appareil (100) selon l'une quelconque des revendications 1 à 15, dans lequel chacun des au moins deux canaux d'écoulement (210, 212 ; 510, 512) s'étend au moins en partie à un angle d'inclinaison (a) par rapport à la première direction, l'angle d'inclinaison tombant dans une plage de 5 degrés à 45 degrés.

17. Appareil (100) selon l'une quelconque des revendications 1 à 15, dans lequel les pores traversants de la membrane (214 ; 514) présentent une taille de pore moyenne tombant dans une plage de 0,2 µm à 10 µm.

18. Appareil (100) selon l'une quelconque des revendications 1 à 17, dans lequel chacun des au moins deux canaux d'écoulement (210, 212 ; 510, 512) dans au moins un module (106) de culture cellulaire de la pluralité de modules (106) de culture cellulaire présente une hauteur de canal variable et une largeur de canal variable qui augmentent vers la seconde chambre (208 ; 508).

19. Appareil (100) selon la revendication 18, dans lequel la largeur de canal variable augmente progressivement vers la seconde chambre (208 ; 508).

20. Appareil (100) selon l'une quelconque des revendications 1 à 19, dans lequel chacun des au moins deux canaux d'écoulement (210, 212 ; 510, 512) dans au moins un module (106) de culture cellulaire de la pluralité de modules (106) de culture cellulaire comprend une première portion de canal (300) et une seconde portion de canal (302), la première portion de canal (300) s'étendant depuis la sortie (218, 222 ; 518, 522) de la partie inférieure de l'un des au moins deux réservoirs (202, 204 ; 502, 504) de milieu de culture et étant parallèle à la première direction, la seconde portion de canal (302) reliant la première portion de canal (300) à l'un des au moins deux trous latéraux de la seconde chambre (208 ; 508), la seconde portion de canal (302) étant inclinée par rapport à la première direction.

21. Incubateur (600) de culture cellulaire comprenant :
l'appareil (100) de culture cellulaire selon l'une quelconque des revendications 1 à 20 ; et une station de pipetage (602) configurée pour alimenter en milieu de culture chacun des au moins deux réservoirs (202, 204 ; 502, 504) de milieu de culture dans chaque module (106) de culture cellulaire de la pluralité de modules (106) de culture cellulaire.

22. Procédé (700) de culture cellulaire en utilisant l'appareil (100) de culture cellulaire selon l'une quelconque des revendications 1 à 20, le procédé (700) comprenant :
(a) la fourniture (S702) du milieu de culture à l'un des au moins deux réservoirs (202, 204 ; 502, 504) de milieu de culture dans chaque module (106) de culture cellulaire de la pluralité de modules (106) de culture cellulaire ; et
(b) le fait d'amener (S704), par l'unité d'entraînement en écoulement (104), le milieu de culture à s'écouler depuis ledit un des au moins deux réservoirs (202, 204 ; 502, 504) de milieu de culture vers le reste des au moins deux réservoirs (202, 204 ; 502, 504) de milieu de culture dans chaque module (106) de culture cellulaire de la pluralité de modules (106) de culture cellulaire pendant une période de temps prédéfinie, la période de temps prédéfinie étant sélectionnée sur la base du milieu de culture.

23. Procédé (700) selon la revendication 22, comprenant en outre, pendant l'étape (b), l'acheminement d'un autre milieu de culture via la première chambre (206 ; 506) vers la membrane (214 ; 514) dans au moins un module (106) de culture cellulaire de la pluralité de modules (106) de culture cellulaire.

24. Procédé (700) selon la revendication 22 ou revendication 23, dans lequel l'étape (b) comprend l'application d'une pression positive aux au moins deux réservoirs (202, 204 ; 502, 504) de milieu de culture dans chaque module (106) de culture cellulaire de la pluralité de modules (106) de culture cellulaire pendant la période de temps prédéfinie.

25. Procédé (700) selon la revendication 22 ou revendication 23, dans lequel l'étape (b) comprend l'application d'une pression positive audit un des au moins deux réservoirs (202, 204 ; 502, 504) de milieu de culture dans chaque module (106) de culture cellulaire de la pluralité de modules (106) de culture cellulaire pendant la période de temps prédéfinie, tout en capsulant le reste des au moins deux réservoirs (202, 204 ; 502, 504) de milieu de culture.

26. Procédé (700) selon la revendication 22 ou revendication 23, dans lequel les au moins deux réservoirs (202, 204 ; 502, 504) de milieu de culture dans chaque module (106) de culture cellulaire de la pluralité de modules (106) de culture cellulaire comprennent un premier réservoir (202 ; 502) de milieu de culture et un second réservoir (204 ; 504) de milieu de culture, et dans lequel l'étape (b) comprend :
- l'application d'une première pression positive au premier réservoir (202 ; 502) de milieu de culture dans chaque module (106) de culture cellulaire de la pluralité de modules (106) de culture cellulaire pendant l'intervalle de temps prédéfini, tout en capsulant le second réservoir (204 ; 504) de milieu de culture ; et
- l'application d'une seconde pression positive au second réservoir (204 ; 504) de milieu de culture dans chaque module (106) de culture cellulaire de la pluralité de modules (106) de culture cellulaire pendant l'intervalle de temps prédéfini, tout en capsulant le premier réservoir (202 ; 502) de milieu de culture.

27. Procédé (800) de culture cellulaire en utilisant l'appareil (100) de culture cellulaire selon l'une quelconque des revendications 1 à 20, le procédé (800) comprenant :
(a) la fourniture (S802) du milieu de culture à un ou plusieurs des au moins deux réservoirs (202, 204 ; 502, 504) de milieu de culture dans chaque module (106) de culture cellulaire de la pluralité de modules (106) de culture cellulaire ;
(b) le fait d'amener (S804), par l'unité d'entraînement en écoulement (104), le milieu de culture à s'écouler vers la seconde chambre (208 ; 508) à partir desdits un ou plusieurs des au moins deux réservoirs (202, 204 ; 502, 504) de milieu de culture dans chaque module (106) de culture cellulaire de la pluralité de modules (106) de culture cellulaire ;
(c) la mise en place (S806) de l'appareil (100) de culture cellulaire dans une position inversée ; et
(d) l'incubation (S808) de l'appareil (100) de culture cellulaire dans la position inversée pendant une période de temps prédéfinie, la période de temps prédéfinie étant sélectionnée sur la base du milieu de culture.

28. Procédé (800) selon la revendication 27, comprenant en outre, pendant l'étape (b), l'acheminement d'un autre milieu de culture via la première chambre (206 ; 506) vers la membrane (214 ; 514) dans au moins un module (106) de culture cellulaire de la pluralité de modules (106) de culture cellulaire.

29. Procédé (800) selon la revendication 28, dans lequel le milieu de culture comprend des cellules endothéliales vasculaires cérébrales humaines et ledit autre milieu de culture comprend des astrocytes humains.
